# EUROPÄISCHE PATENTSCHRIFT

(11) **EP 2 357 023 B1**
(45) Veröffentlichungstag und Bekanntmachung des Hinweises auf die Patenterteilung: **07.12.2011**
(21) Anmeldenummer: 10153447.7
(22) Anmeldetag: 12.02.2010
(51) Int. Cl.: A61N 5/10

(54) **Gerät und System zum Zusammenfügen von Kettenbestandteilen zu einer Kette umfassend radioaktive Strahlenquellen**
Device and system for joining together chain components comprising radioactive radiation sources to form a chain
Appareil et système d'assemblage de composants de chaînes pour une chaîne contenant des sources de rayonnement radioactives

(43) Veröffentlichungstag der Anmeldung: 17.08.2011
(73) Patentinhaber: Eckert & Ziegler Bebig GmbH, 13125 Berlin (DE)
(72) Erfinder: Hentrich, Dr. Axel, 13125, Berlin (DE); Lederer, Christoph Dipl.-Ing., 10437, Berlin (DE)
(74) Vertreter: Gulde Hengelhaupt Ziebig & Schneider

(56) Entgegenhaltungen:
- EP-A1- 1 070 519
- WO-A1-2005/002670
- US-A1- 2008 161 635

## Beschreibung

Die Erfindung betrifft ein Gerät und ein System zum Zusammenfügen von Kettenbestandteilen zu einer Kette, die radioaktive Strahlenquellen umfasst. Die Verwendung des Gerätes ist für die Herstellung von Ketten aus Strahlenquellen und Abstandshaltern zur Behandlung von Prostatakarzinomen konzipiert.

### Stand der Technik

Es ist bekannt, Tumorpatienten, insbesondere Prostatatumorpatienten eine Kette bestehend aus radioaktiven Strahlenquellen, sogenannten Seeds einzupflanzen. Dazu sind sowohl Einzelstrahlenquellen als auch eine vorkonfigurierte Seedketten aus z.B. abwechselnd einer radioaktivem Strahlenquelle und einem nichtaktiven Abstandshalter oder Spacer bekannt. Die Implantation von Einzelstrahlenquellen erlaubt unter Verwendung des entsprechenden Instrumentariums eine individuelle Positionierung der einzelnen Strahlenquellen. Bedingt durch die entzündliche Vergrößerung der Prostata nach der Implantation gefolgt von einem Abschwellen kann es jedoch unter Umständen zu einer Migration der Strahlenquellen bzw. zu einer Verschiebung ihrer planbestimmten Position kommen. Eine Strahlenquellenkette verhindert diese Positionsverschiebung, da sie die Einzelimplantate miteinander verbindet.

Neue medizinische Untersuchungen zeigen, dass die Einhaltung eines individuell auf den Patienten abgestimmter Bestrahlungsplanes die besten Resultate bei der Tumorbehandlung erzielt. Diesen einzuhalten erfordert die auf jeden Patienten angepasste genaue Positionierung der einzelnen radioaktiven Strahlenquellen.

Aus WO 2009/005528 A1 ist ein Gerät zum Zusammenfügen von frei konfigurierbaren Seed-Spacer-Ketten aus insgesamt fünf verschiedenen Magazinen mit nebeneinander angeordneten Implantaten bekannt. Die Ketten werden durch Ineinanderschieben der Einzelimplantate miteinander verbunden. Die Auswahl der Magazine erfolgt dabei durch ein Verschieben eines Schlittens quer zum Arbeitskanal. Dieser Schlitten dient der Aufnahme der Einzelmagazine. Eine solche Auswahl mit einem Schlitten ist jedoch sehr umständlich, da immer wieder der Schlitten mit den Magazinen und einem Arbeitskanal zur Aufnahme der ausgewählten Kettenbestandteile ausgerichtet werden muss. Dabei kann es auch leicht zum Verklemmen von Bestandteilen des Geräts kommen. Da ferner die Magazine aus transparentem Polymer bestehen, sind sie nur in der Verpackung bzw. nach Einstecken in das Beladungsgerät strahlensicher. Der Inhalt der Magazine ist auch aus diesem Grund auf ca. 20 Strahlenquellen begrenzt.

US 7,025,717 B2 beschriebt ein entsprechendes Gerät, welches aus zwei Magazinen mit nebeneinander angeordneten Implantaten das frei konfigurierbare Zusammenstellen von Strahlenquellen und Abstandshaltern erlaubt. Die Implantate werden hierbei jedoch nicht zu festen Ketten verbunden.

US 6,454,696 B1 beschreibt direkt hintereinanderliegende Rundmagazine, in denen die Strahlenquellen und Abstandshalter konzentrisch zum Drehpunkt der Magazine angeordnet sind. Nachteilig an der Anordnung ist jedoch, dass die Einzelimplantate des hinteren Magazins bei dieser Erfindung konstruktionsbedingt durch das vordere Magazin durchgeschoben werden müssen. Das Hindurchschieben durch verschiedene Magazine kann jedoch zu einem Verklemmen der Vorrichtung führen, welches dann manuell wieder beseitigt werden muss. Ein Hindurchschieben von Kettenbestandteilen durch verschiedene Magazine ist auch aus US 6,358,195 B1 bekannt.

### Offenbarung der Erfindung

Aufgabe der Erfindung ist es daher, ein Gerät und ein System zum Zusammenstellen von Strahlenquellenketten bereitzustellen, welche dem behandelnden OP-Personal die Möglichkeit geben, unter Einhaltung eines individuellen Bestrahlungsplanes die Seedimplantation anwenderfreundlich durchzuführen. Anwenderfreundlich ist hierbei bezogen auf die Gerätebedienung sowie auf den optimalen Strahlenschutz für den Anwender zu verstehen.

Dementsprechend wird ein Gerät zum Zusammenfügen von Kettenbestandteilen zu einer Kette vorgeschlagen, bei dem mindestens ein Kettenbestandteil aus mindestens einer radioaktiven Strahlenquelle besteht, welches ein Gehäuse umfasst sowie einen Arbeitskanal, der sich entlang einer ersten Achse des Gehäuses erstreckt, eine Beladungseinheit, verbunden mit dem Arbeitskanal und umfassend mindestens zwei Aufnahmeeinrichtungen für Magazine von Kettenbestandteilen, wobei mindestens eine Aufnahmeeinrichtung geeignet ist, ein Strahlenquellenmagazin aufzunehmen, sowie mindestens ein Mittel zum Auswerfen der Kettenbestandteile aus den aufgenommenen Magazinen, und eine Fügeeinheit zum Zusammenfügen von Kettenbestandteilen. Dabei sind die mindestens zwei Aufnahmeeinrichtungen derart angeordnet, dass in ihnen aufgenommene Magazine für Kettenbestandteile beabstandet entlang der ersten Achse vertikal über dem Arbeitskanal angeordnet sind. Mit anderen Worten sind die Aufnahmeeinrichtungen selbst beabstandet entlang der ersten Achse vertikal über dem Arbeitskanal angeordnet.

Die beschriebene Erfindung soll es den Anwendern ermöglichen, aus bereitgestellten Strahlenquellen- bzw. Abstandshaltermagazinen Strahlenquellen und Abstandshalter, im Weiteren zusammenfassend als Implantate bezeichnet, als Kettensegmente in nahezu freier Konfiguration zusammenzustellen und zu fügen. Die aus dem Stand der Technik bekannte Limitierung kann somit weitestgehend vermieden werden. Das Bedienen dieses Geräts erfolgt einfach und intuitiv. Das beschriebene Gerät verfügt über einen zentralen Arbeitskanal, in den die Implantate von oben aus den Magazinen abgelegt werden. Die Ablage der Implantate von oben in den zentralen Arbeitskanal ermöglicht ein Aufreihen der Magazine entlang der Gerätelängsachse. Implantate aus dem hinteren Magazin müssen hierbei nicht durch das vordere Magazin durchgeschoben werden, was zu Verklemmungen führen kann. Diese Anordnung der Magazine verhindert zur Magazinauswahl ein zusätzliches aktives Verschieben einer Magazinaufnahme senkrecht oder in einem anderen Winkel zur Längsachse. Darüberhinaus wird durch den Einsatz von nur zwei Magazinen ein Verwechseln der Magazine beim Auslösen der Implantate erschwert.

Der Mechanismus zum Auswerfen ist bevorzugt ein Hebelmechanismus und erlaubt dabei, dass ein Auswerferhebel eines Magazins nach Implantatfreigabe zwar wieder in seine Ausgangsposition zurückgeht, jedoch eine erneute Auslösung erst nach Entfernen des zuvor ausgelösten Implantats erfolgen kann.

Bevorzugt ist die zweite Aufnahmeeinrichtung eingerichtet, ein Abstandshaltermagazin mit Abstandshaltern aufzunehmen.

Die Fügeeinheit umfasst in einem Ausführungsbeispiel einen Fügebereich und eine Sichteinheit, so dass ein Zusammenfügen der Kette von außen betrachtbar ist.

Vorteilhafterweise ist dadurch das Konfigurieren der einzelnen Implantate visuell verfolgbar und bis zum endgültigen Fügen der Kettenglieder über einfaches Öffnen einer Deckklappe korrigierbar.

Die Sichteinheit ist bevorzugt eine indirekte Sichteinheit ist, so dass die Kette indirekt betrachtbar ist. Besonders bevorzugt umfasst die Sichteinheit eine Spiegel-Linseneinheit.

Ferner kann das Gerät eine Klappe zum Öffnen des Gehäuses im Fügebereich der Fügeeinheit umfassen.

Bevorzugt ist ein Nadelhalter an einem Ende des Arbeitskanals angeordnet und zwischen Nadelhalter und Fügeeinheit ist eine erste Sperre angeordnet.

Die Fügeeinheit umfasst ferner einen Mandrel, der im Arbeitskanal entlang der ersten Achse verschiebbar ausgebildet ist. Der Mandrel ist bevorzugt über einen an dem Gehäuse verschiebbar gelagerten äußeren Griff verschiebbar ausgebildet.

Die Bauweise des Geräts mit einem Arbeitskanal unter den Magazinen und einem Mandrel im Arbeitskanal vermeidet ein durch Fehlbedienung verursachtes Verbiegen des Mandrels.

Die Fügeeinheit kann ferner eine Magnetkupplung umfassen, derart, dass der äußere Griff und der Mandrel bei Überschreitung eines Kraftschwellenwertes voneinander trennbar ausgebildet sind.

Der Mandrel und das mindestens eine Mittel zum Auswerfen von Kettenbestandteilen sind bevorzugt miteinander über eine zweite Sperre derart gekoppelt, dass nach einmaliger Betätigung des Mittels zum Auswerfen keine zweite Betätigung des Mittels zum Auswerfen erfolgen kann, bevor eine Verschiebung des Mandrels vorgenommen wurde.

Die Aufnahmeeinrichtungen umfassen bevorzugt Sicherungsmechanismen zum Einrasten, Sichern und Herauslösen von Magazinen mit Kettenbestandteilen.

Das Gerät kann eine dritte Sperre umfassen, die im verriegelten Zustand eine Verschiebung des Mandrels nur bis zu Punkt ermöglicht, bei dem ein Zusammenfügen der Kettenbestandteile im Arbeitskanal noch nicht erfolgt. Die dritte Sperre ist bevorzugt mit der ersten Sperre zwischen Nadelhalter und Fügeeinheit verknüpft, so dass nur mit gelöster dritter Sperre auch die erste Sperre im Nadelhalter entriegelbar ist.

Die Aufnahmeeinrichtungen können Elemente zur Kodierung enthalten, die mit entsprechenden Kodierungselementen eines Magazins zusammenwirkbar ausgebildet sind, sodass eine Aufnahmeeinrichtung nur für eine spezielle Art von Magazin verwendbar ist.

Es wird ferner ein System zum Zusammenfügen von Kettenbestandteilen zu einer Kette mit radioaktiven Strahlenquellen vorgeschlagen, welches ein Gehäuse umfasst sowie einen Arbeitskanal, der sich entlang einer ersten Achse des Gehäuses erstreckt, eine Beladungseinheit, verbunden mit dem Arbeitskanal und umfassend mindestens zwei Aufnahmeeinrichtungen für Magazine von Kettenbestandteilen sowie mindestens ein Mittel zum Auswerfen der Kettenbestandteite aus den aufgenommenen Magazinen, ein erstes Magazin für radioaktive Strahlenquellen, angeordnet in einer der mindestens zwei Aufnahmeeinrichtungen, ein zweites Magazin für weitere, von den Strahlenquellen verschiedene Kettenbestandteile, angeordnet in der anderen der mindestens zwei Aufnahmeeinrichtungen, und eine Fügeeinheit zum Zusammenfügen von Kettenbestandteilen. Dabei führt der Arbeitskanal nicht durch die Magazine hindurch und das erste Magazin für radioaktive Strahlenquellen sowie das zweite Magazin für weitere Kettenbestandteile sind beabstandet entlang der ersten Achse vertikal über dem Arbeitskanal derart angeordnet, dass Kettenbestandteile nach unten in den Arbeitskanal ausgeworfen werden.

### Kurze Beschreibung der Zeichnungen

Ausführungsbeispiele der Erfindung werden anhand der Zeichnungen und der nachfolgenden Beschreibung näher erläutert. Es zeigen:
- Fig. 1: eine Draufsicht auf ein erfindungsgemäßes Magazin für Kettenbestandteile einer Kette mit radioaktiven Strahlenquellen,
- Fig. 2: eine Draufsicht auf ein erfindungsgemäßes Magazin für Kettenbestandteile einer Kette mit radioaktiven Strahlenquellen ohne Deckel,
- Fig. 3: eine Draufsicht auf ein erfindungsgemäßes Magazin für Kettenbestandteile einer Kette mit radioaktiven Strahlenquellen ohne Deckel, ohne Aufnahmevorrichtung für Kettenbestandteile und ohne Zahnkranz,
- Fig. 4: Kernbestandteile des Magazins ohne Gehäuse,
- Fig. 5: einen vertikalen Schnitt durch das Magazin der Fig. 1,
- Fig. 6-11: den erfindungsgemäßen Auswerfmechanismus in verschiedenen Schritten,
- Fig. 12: den erfindungsgemäßen Blockiermechanismus in den Magazinen
- Fig. 13: zusammenfügbare Abstandshalter und Strahlenquellen,
- Fig. 14: eine perspektivische Ansicht eines erfindungsgemäßen Gerätes zum Fügen und Konfektionieren von Ketten, die Strahlenquellen beinhalten;
- Fig. 15: eine Seitenansicht des erfindungsgemäßen Gerätes zum Fügen und Konfektionieren von Ketten der Fig. 14,
- Fig. 16: einen perspektivischen Querschnitt durch das erfindungsgemäße Gerät entlang der Achse A -A in Fig. 15, gesehen entgegen der Richtung der Pfeile der Fig. 15,
- Fig. 17: einen Querschnitt durch das erfindungsgemäße Gerät entlang der Achse A-A in Fig. 15, gesehen in Richtung der Pfeile der Fig. 15,
- Fig. 18: eine erste Ansicht des erfindungsgemäßen Gerätes ohne Gehäuse,
- Fig. 19: eine zweite Ansicht des erfindungsgemäßen Gerätes ohne Gehäuse,
- Fig. 20: einen Querschnitt durch das Gerät zum Fügen durch ein Abstandshaltermagazin;
- Fig. 21: eine Vergrößerung der Fig. 20 im unteren Bereich des Abstandshaltermagazins;
- Fig. 22: einen Längsschnitt durch den vorderen Bereich des erfindungsgemäßen Gerätes zum Fügen im Bereich des Nadelhalters.

### Detaillierte Beschreibung der Zeichnungen

Die vorliegende Erfindung beschreibt ein Gerät und ein System zum Zusammenfügen von Kettenbestandteilen zu einer Kette, die radioaktive Strahlenquellen umfasst. Die Verwendung des Gerätes ist für die Herstellung von Ketten aus Strahlenquellen und Abstandshaltern zur Behandlung von Prostatakarzinomen konzipiert. Es kann aber auch zum Herstellen von Ketten mit Strahlenquellen zur Behandlung von Mammalkarzinomen verwendet werden. Ebenso ist aber auch das Laden von Nadeln mit Titanseeds möglich. Die Abstandshalter und Strahlenquellen weisen im Folgenden Verbindungselemente auf, damit aus Ihnen durch mechanische Verbindung Ketten gefertigt werden können.

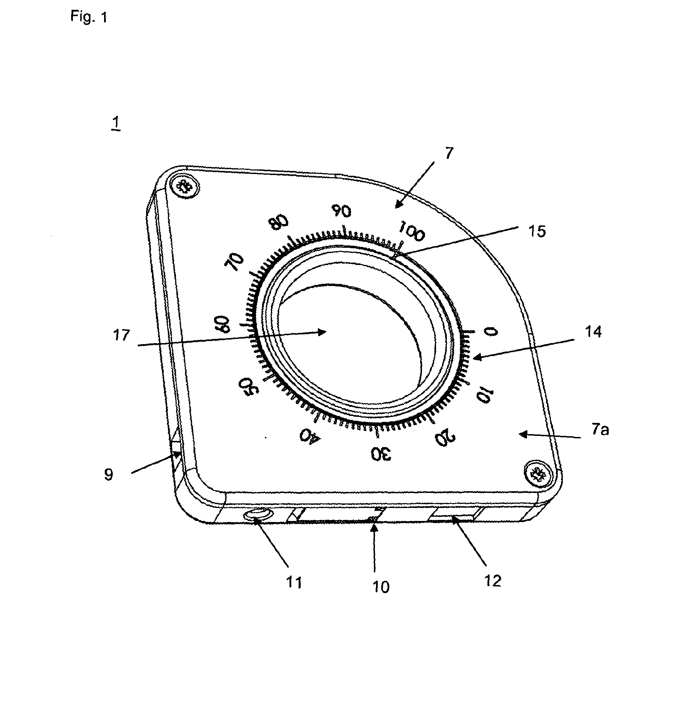

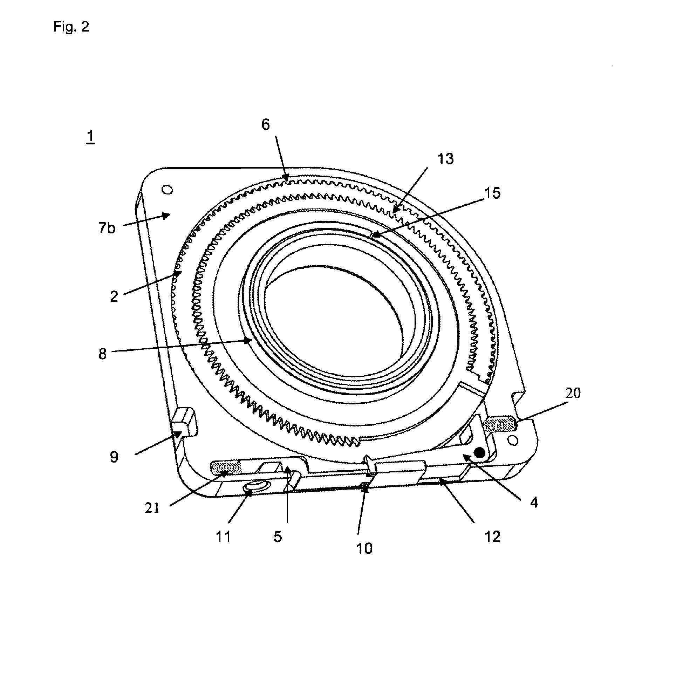

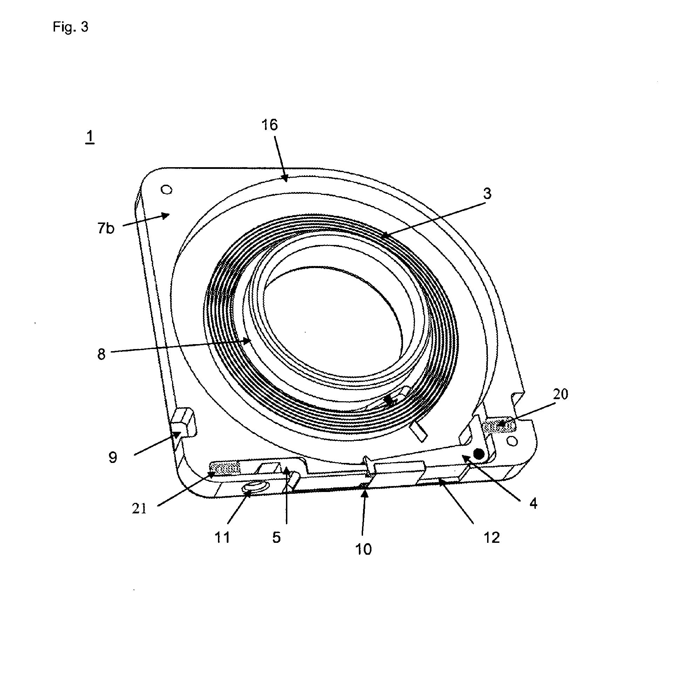

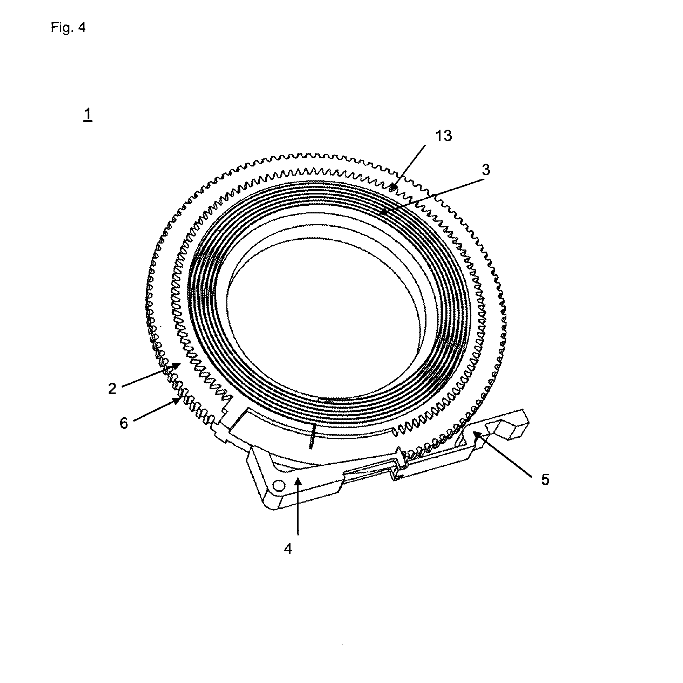

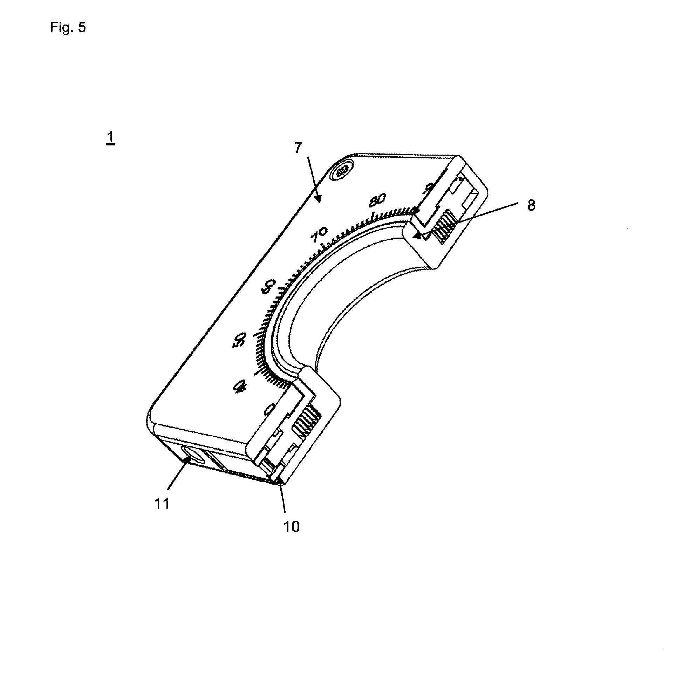

Fig. 1 zeigt eine Draufsicht auf ein erfindungsgemäße Magazin 1 für Kettenbestandteile einer Kette mit radioaktiven Strahlenquellen. In Fig. 2 ist ein Magazin ohne Deckel 7a gezeigt. In Fig. 3 fehlen zudem eine Aufnahmevorrichtung für Kettenbestandteile 2 und ein Zahnkranz 13. Fig. 4 zeigt die Kernbestandteile des Magazins ohne Gehäuse 7 und Fig. 5 zeigt einen vertikalen Schnitt durch das Magazin der Fig. 1.

Wie in Fig. 1 und 2 gezeigt, umfasst ein erfindungsgemäßes Magazin 1 ein Gehäuse 7 bestehend aus einem Deckel 7a und einer Gehäuseschale 7b. Der Deckel umfasst eine Anzeige 14 zum Anzeigen des Füllstandes des Magazins in Verbindung mit einer Markierung 15. Die Markierung ist bevorzugt auf einem inneren Lagerring Mittels zur Aufnahme von, Kettenbestandteilen 2 angeordnet.

An der Peripherie des Magazins 1, bevorzugt an der unteren Seitenwand des Gehäuses 7 ist eine Kodierungsbohrung 11, ein Auswurf 10 und eine erste Öffnung 12 angeordnet. Es ist bevorzugt nur genau ein Auswurf bzw. eine Auswurfsöffnung 10 vorgesehen, damit die Strahlenbelastung bei Verwendung von Strahlenquellen als Kettenbestandteile minimal gehalten werden kann. Der Auswurf erfolgt also in derselben oder einer parallelen Ebene, in der sich das Mittel zur Aufnahme von Kettenbestandteilen dreht. Der Auswurf des Kettenbestandteils erfolgt vom Magazin weg. Hat der Kettenbestandteil den Auswurf verlassen, so besteht kein Kontakt mehr zwischen Magazin und Kettenbestandteil. Kodierungsbohrung 11 und/oder Auswurf 10 und/oder erste Öffnung 12 sind bevorzugt getrennt vom Mittel zur Aufnahme von Kettenbestandteilen 2 unterhalb des Mittels zur Aufnahme von Kettenbestandteilen 2 aber in derselben oder eine parallelen Ebene angeordnet.

In einem bevorzugten Ausführungsbeispiel ist das Magazin nur für genau eine Sorte von Kettenbestandteilen konfiguriert, also entweder für Strahlenquellen oder für Abstandshalter. Somit kann keine Verwechslung der ausgeworfenen Kettenbestandteile auftreten.

In Verbindung mit der ersten Öffnung 12 ist ein Auswerferhebel 4 angeordnet, der drehbar im Gehäuse 7 gelagert ist. Der Auswerferhebel 4 ist durch die erste Öffnung 12 von außen betätigbar. In Ruhestellung verschließt der Auswerferhebel 4 den Auswurf 10 von innen. Der Auswurf 10 steht ferner mit einem Verschlussschieber 5 in Verbindung, der durch die Kraft einer zweiten Spannfeder 21 den Auswurf 10 nach außen verschließt. Der Verschlussschieber 5 ist translatorisch verschiebbar, so dass der Auswurf 10 bei Betätigung des Verschlussschiebers 5 freigegeben werden kann. Der Verschlussschieber 5 ist über eine Öffnung 11 oder Kodierungsbohrung 11 zur Freigabe des Auswurfs 10 betätigbar. Ferner weist das Magazin bevorzugt aber nicht beschränkend eine zentrale Öffnung 17 auf. Wie man in Fig. 2 sehen kann, ist in der hinteren Gehäuseschale 7b ein kreisförmiges Mittel zur Aufnahme von Kettenbestandteilen 2, auch im Falle eines Magazins für Strahlenquellen "Seedablage" 2 genannt, drehbar gelagert eingesetzt. Das Mittel zur Aufnahme von Kettenbestandteilen 2 umfasst dabei radial angeordnete Vertiefungen 6, die derart ausgebildet sind, dass sie Kettenbestandteile wie Strahlenquellen 19 oder Abstandshalter 18 aufnehmen und gegen ein Herausfallen lagern können. Die Vertiefungen 6 sind bevorzugt der Form der Kettenbestandteile entsprechend ausgebildet. Besonders bevorzugt sind sie halbkreisförmig auf dem kreisförmigen Rand entlang des Umfangs des Mittels zur Aufnahme von Kettenbestandteilen 2 angeordnet. Mit anderen Worten, dass Mittel zur Aufnahme von Kettenbestandteilen 2 ist einem Zahnrad ähnelnd ausgebildet, umfasst aber anstatt von Zähnen Vertiefungen 6 auf seinem äußeren Rand. Eine Spannfeder 3, bevorzugt eine Konstantkraftfeder 3 erlaubt einen Vortrieb des Mittels zur Aufnahme von Kettenbestandteilen 2 konzentrisch zu ihrem Mittelpunkt. Somit ist keine äußere Antriebskraft des Magazins zum Auswurf eines Kettenbestandteils nötig. Das Magazin ist voll sterilisierbar. Es bedarf zum Auswurf eines Kettenbestandteils lediglich eines Impulses von außen an den Auswerferhebel, damit der Vortrieb im Inneren selbständig vorgenommen werden kann. Als Lagerfläche für das Mittel zur Aufnahme von Kettenbestandteilen 2 dient eine Materialwand des Gehäuses 7 als innerer Lagerring 8. Der innere Lagerring 8 ist bevorzugt um die Öffnung 17 herum ausgebildet. Ferner ist auf dem Mittel zur Aufnahme von Kettenbestandteilen 2 ein Zahnkranz 13, bevorzugt mit einem kleineren Durchmesser als das Mittel zur Aufnahme von Kettenbestandteilen 2, angeordnet. Der Zahnkranz 13 steht dabei in Wirkverbindung zum Mittel zur Aufnahme von Kettenbestandteilen 2 oder direkt zur Konstantkraftfeder 3 und ist ebenfalls drehbar im Gehäuse 7 gelagert. Der Zahnkranz 13 ist fest mit dem Mittel zur Aufnahme von Kettenbestandteilen 2 gegen Verdrehung zueinander verbunden. Er dient der Begrenzung des Vortriebs der Konstantkraftfeder 3 während des Auswurfs, so dass immer nur ein Kettenbestandteil ausgeworfen werden kann. Bevorzugt sind Zahnkranz 13 und Mittel zur Aufnahme von Kettenbestandteilen einstückig ausgebildet.

Vom Zentrum des Magazins 1 nach außen hin besteht das Magazin 1 aus einer zentralen Öffnung 17, einem inneren Lagerring 8, dem Zahnkranz 13, das Mittel zur Aufnahme von Kettenbestandteilen 2 und einer Begrenzung 16 des Mittels zur Aufnahme von Kettenbestandteilen 2. Im vorliegenden Ausführungsbeispiel sind Zahnkranz 13 und das Mittel zur Aufnahme von Kettenbestandteilen 2 in einer Vertiefung in die hintere Gehäuseschale 7b eingelassen.

Die innere Wandung 16 der Vertiefung (s. Fig. 3) dient dabei der Begrenzung des Mittels zur Aufnahme von Kettenbestandteilen 2. Der Abstand zwischen Wandung 16 der Vertiefung oder allgemeiner, der Begrenzung 16 des Mittels zur Aufnahme von Kettenbestandteilen 2 und des Mittels zur Aufnahme von Kettenbestandteilen 2 selbst ist so dimensioniert, dass die Kettenbestandteile in den Vertiefungen 6 ohne herauszufallen führbar gelagert werden können. Die Kettenbestandteile werden dabei auf einer Kreisbahn zum Auswurf 10 geführt. Unter dem Mittel zur Aufnahme von Kettenbestandteilen 2 ist die Spannfeder 3 gelagert, wie in Fig. 3 gezeigt.

Die Feder 3 und damit das Mittel zur Aufnahme von Kettenbestandteilen 2 werden durch den Auswerferhebel 4 wie weiter unten beschrieben gehemmt. Die Hemmung der Feder 3 und des Mittels zur Aufnahme von Kettenbestandteilen 2 gestattet immer nur das Freisetzen eines Implantats bzw. Kettenbestandteils aus dem Magazin pro Betätigung des Magazins. Die Hemmung erfolgt zum einen durch den Formschluss des ersten Implantats an dem Auswerferhebel 4 und zum anderen durch die Taktverzahnung des oberen Zahnkranzes 13. Der Auswerferhebel 4 unterstützt außerdem das Freisetzen des Implantats, indem er es aktiv in einen Arbeitskanal schiebt. Der Auswerferhebel 4 ist bevorzugt federngelagert und wird durch einen entsprechenden Hebelmechanismus im später beschriebenen Gerät zum Beladen 101 betätigt. Die Implantate werden aus der Öffnung 10 an der Magazinunterseite in einen Arbeitskanal freigesetzt. Bevorzugt sind die Magazine 1 mechanisch codiert und farblich markiert. Die farbliche Markierung kann durch Färbung des Mittels zur Aufnahme von Kettenbestandteilen 2 erfolgen, die den beweglichen Teil der Skala darstellt. Die Anzahl der verbrauchten Strahlenquellen und Abstandshalter können direkt an den Magazinen abgelesen werden.

Im Folgenden wird die Beschreibung des Magazins beispielhaft anhand eines Strahlenquellenmagazins beschrieben. Dies erfolgt jedoch nicht beschränkend. Die Ausführungen gelten auch für Abstandshaltermagazine, es sei denn etwas Gegenteiliges ist explizit erwähnt.

Zur Montage des Magazins 1 wird zuerst die Spannfeder 3 an die hintere Gehäusehälfte montiert und dann gemeinsam mit dem Mittel zur Aufnahme von Kettenbestandteilen 2 in die hintere Gehäuseschale 7b eingesetzt. Anschließend werden ein Auswerferhebel 4 und ein Verschlussschieber 5 eingesetzt und jeweils verspannt, bevorzugt mit Druckfedern 20 und 21, wie in Fig. 3 gezeigt. Hierbei ist der Auswerferhebel 4 über einen Stift rotatorisch gelagert und der Verschlussschieber 5 kann in einer Nut in der Gehäuseschale 7b translatorisch verschoben werden.

Nach der Montage des Magazins 1 ohne Deckel 7a werden in den halbkreisförmigen Vertiefungen 6 an der Stirnseite des Mittels zur Aufnahme von Kettenbestandteilen 2 die Strahlenquellen 19 (oder Abstandshalter 18) parallel zur Achse des Mittels zur Aufnahme von Kettenbestandteilen 2 positioniert. Hierbei muss vor dem Beladen des Mittels zur Aufnahme von Kettenbestandteilen 2 die Spannfeder 3, bevorzugt eine Konstantkraftfeder 3 gespannt werden. Dies erfolgt durch Verdrehen des Mittels zur Aufnahme von Kettenbestandteilen 2 in Spannrichtung der Spannfeder 3. Ein Blockieren gegen das Entspannen der Spannfeder 3 erfolgt durch das Setzen der ersten Strahlenquellen 19. Die weiteren Strahlenquellen 19 werden anschließend eingesetzt. Im späteren Betrieb des Magazins 1 dient die jeweilig erste Strahlenquellen 19 vor ihrer Freigabe immer als Blockierelement oder Mittel zum Blockieren gegen das Entspannen der Spannfeder 3. Nach dem Befüllen wird das Magazin 1 mit einem Deckel 7a versehen. Ein innerer Lagerring 8 des Mittels zur Aufnahme von Kettenbestandteilen 2 ist verbreitert und dient mit einer Markierung 15 versehen gemeinsam mit dem Magazindeckel 7a als Inhaltsanzeige für die noch in den Magazinen 1 verbliebenen Strahlenquellen 19 bzw. Abstandshalter 18.

Das Magazin 1 für die Lagerung von Abstandshaltern 18 ist mit Ausnahme des Verschlussschiebers 5 und der entsprechenden Ausfräsung in der hinteren Gehäuseschale 7b baugleich mit dem Strahlenquellenmagazin. Daher erfolgen die Montage und das Befüllen beider Magazintypen analog. Der Verschlussschieber 5 kann im Abstandshaltermagazin ebenfalls angeordnet sein. Er ist aber nicht zwingend notwendig, da die Abstandshalter nicht radioaktiv sind. Der Verschlussschieber 5 des Strahlenquellenmagazins dient der Abschirmung der Strahlung nach Außen, bevor das Magazin in ein Gerät 101 zum Befüllen eingeführt wird.

Mindestens eine, bevorzugt beide schmalen Seitenflächen eines Magazins 1 sind mit Sicherungsnuten 9 versehen. Diese dienen nach dem Einsetzen der Magazine 1 in ein Gerät 101 zur Konfektionierung von Strahlenquellenkette als Sicherung gegen ein Herausfallen aus dem Gerät sowie als Positionierhilfe gegenüber einem Arbeitskanal im Gerät 101.

Im Mittenbereich der Unterfläche des Strahlenquellenmagazins befindet sich eine Öffnung zum Auswurf der Implantate 10. Auf der einen Seite dieser Öffnung 10 ist die Seitenfläche mit einer Kodierungsbohrung 11 versehen. Beim Einsetzen des Strahlenquellenmagazins in ein Gerät zum Fügen von Strahlenquellenketten 101 wird durch einen Kodierungsdorn im Gerät 101 der Verschlussschieber 5 so verschoben, dass er die Auswurföffnung 10 freigibt. Diese Funktion existiert bevorzugt im Abstandshaltermagazin nicht, da der Verschlussschieber 5 verhindern soll, einen Operateur radioaktiver Strahlung aus den Strahlenquellen 19 auszusetzen. Dies ist bei Abstandshaltern 18 nicht notwendig, da sie nicht radioaktiv sind. Natürlich kann aber dennoch der Schieber 5 vorgesehen sein.

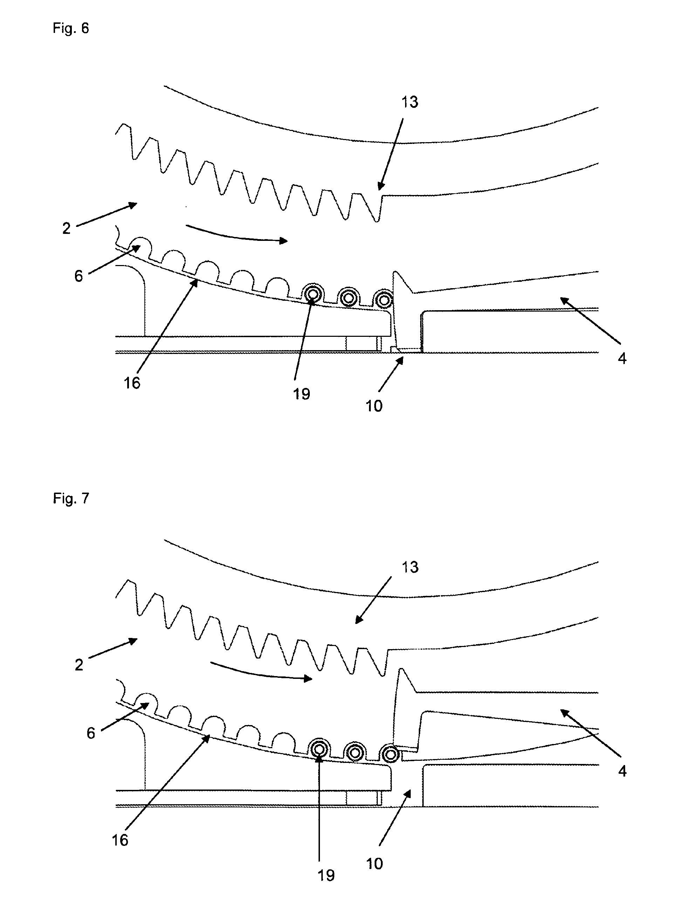

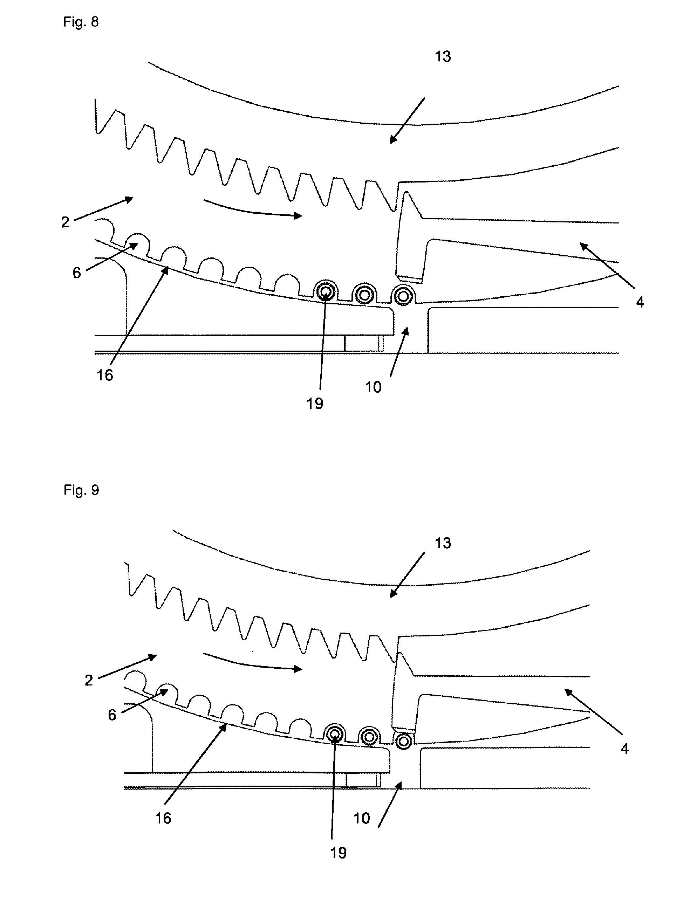

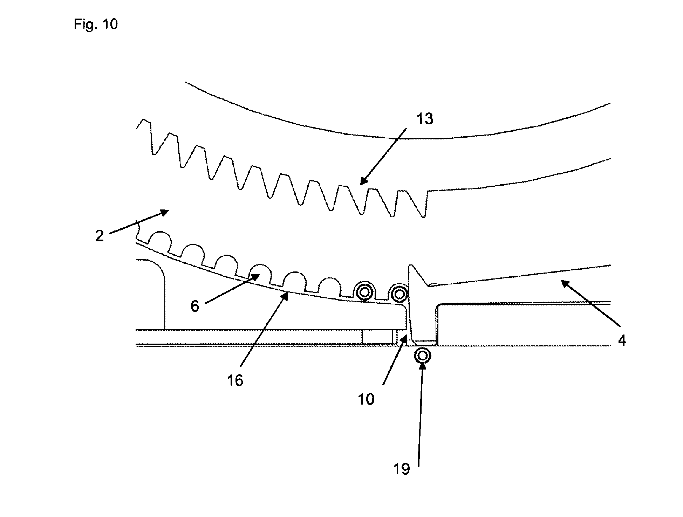

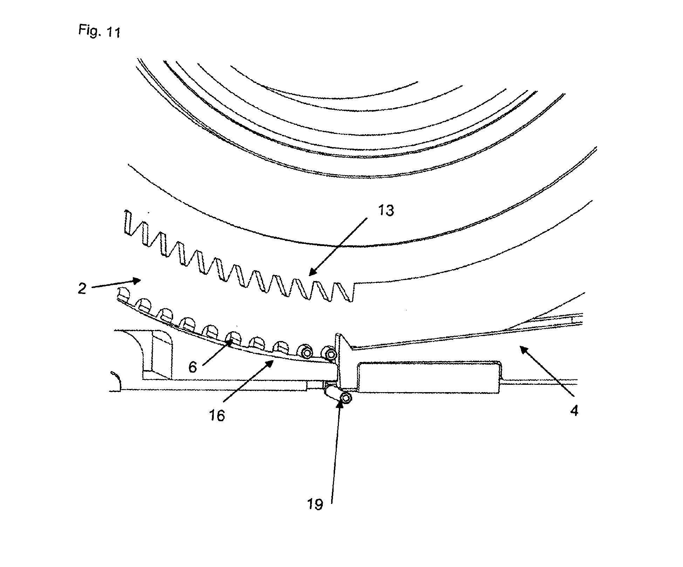

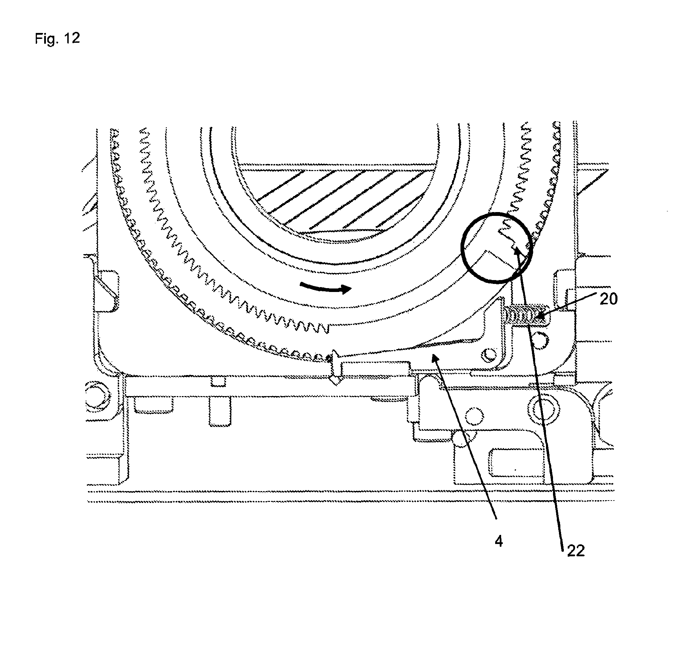

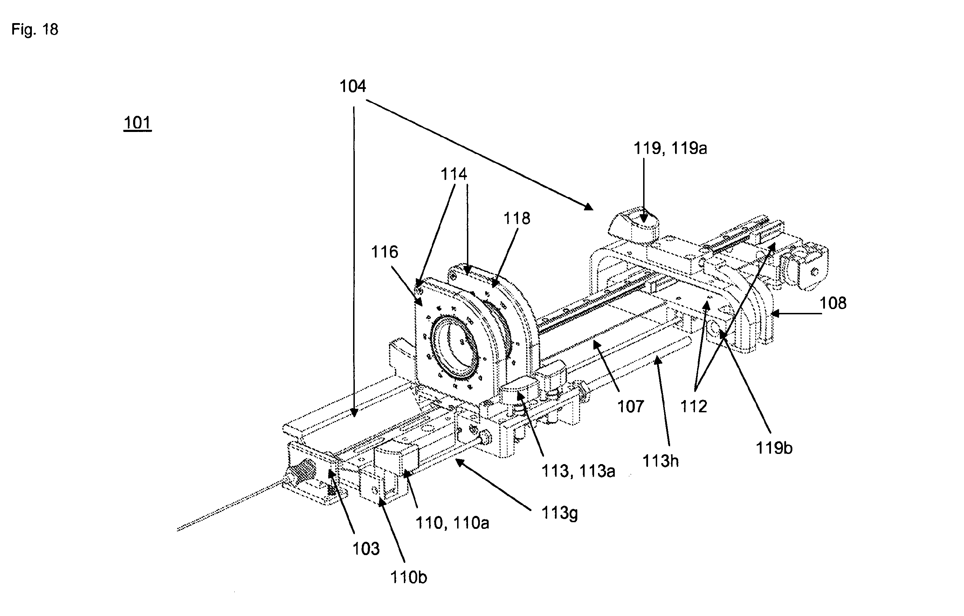

Durch eine zweite Öffnung 12 auf der anderen Seite der Auswurföffnung 10 lässt sich der Auswurfhebel 4 durch einen entsprechenden Hebelmechanismus 113 im Gerät 101 betätigen. Der Auswurfmechanismus ist in den Figuren 6 bis 11 in einzelnen Teilschritten dargestellt. Zunächst ist wie in Fig. 6 dargestellt der Auswurf 10 durch den Auswurfhebel 4 physisch blockiert. Der Auswurfhebel 4 greift in dieser Position nicht in den Zahnkranz 13 ein. Das Auswerfen jeweils einer Strahlenquelle 19 bzw. eines Abstandshalters 18 aus den Magazinen 1 erfolgt durch ein Betätigen der äußeren Fläche des Auswurfhebels 4. Dadurch gleitet der Auswurfhebel 4 an der ersten Strahlenquelle 19 bzw. dem ersten Abstandshalter 18 nach oben hin ab (Fig. 7). Die erste Strahlenquelle 19 bzw. Abstandshalter 18 verliert seine Blockierfunktion der Fig. 6, das Mittel zur Aufnahme von Kettenbestandteilen 2 bewegt sich getrieben durch die gespannte Feder 3 konzentrisch zur Lochöffnung 17 oder Mitte des Magazins 1. Diese Bewegung wird jedoch durch das Eingreifen des Auswurfhebels 4 in die Taktverzahnung des oberen Zahnkranzes 13 des Mittels zur Aufnahme von Kettenbestandteilen 2 im Rahmen der weiteren Bewegung blockiert (Fig. 8, 9). Durch diese Drehung des Mittels zur Aufnahme von Kettenbestandteilen 2 wird die erste Strahlenquelle (bzw. Abstandshalter) und nur diese in eine Position befördert, die über der Auswurföffnung 10 liegt (Fig. 9). Die erste Strahlenquelle wird dann durch den Auswerferhebel 4 nach unten hinten, bevorzugt in einen Arbeitskanal (nicht gezeigt) unter dem Auswurf 10 zum Zusammenfügen der Kettenbestandteile freigesetzt (Fig. 10 und 11). Nach dem Betätigen des Auswurfhebels 4 fällt dieser wieder in seine vorherige Position zurück und blockiert wiederum den Auswurf 10 wie in Fig. 6. Somit wird die weitere Drehung des Mittels zur Aufnahme von Kettenbestandteilen 2 erneut durch das Anliegen der nächsten ersten Strahlenquelle 19 bzw. des nächsten ersten Abstandshalters 18 am Auswurfhebel 4 verhindert (Fig. 11). Durch diesen Mechanismus wird eine schrittweise Drehung des Mittels zur Aufnahme von Kettenbestandteilen 2 und ein Einzelabwurf der Strahlenquellen 19 bzw. Abstandshalter 18 in einen Arbeitskanal realisiert. Ein Auslösen der Hebel 4 zur Magazinbetätigung bei leeren Magazinen 1 ist nicht möglich.. Dies ist in Fig. 12 dargestellt. Mit einem Pfeil ist die Drehrichtung des Mittels zur Aufnahme von Kettenbestandteilen 2 dargestellt. Das letzte Implantat im Magazin ist an der mit einem Kreis markierten Stelle angeordnet. Nach dem Auswurf des letzten Implantats aus dem Magazin, fällt der Hebel 4 gezwungen durch die Antriebsfeder 3 und die Hebelfeder, die erste Druckfeder 20, in die eingekreiste Einkerbung des Blockierelementes 22. Das Blockierelement 22 bietet keinen Platz für eine Auslenkung des Hebels 4. Das Betätigen der Tasten 113a des Hebelmechanismus 113 zum Auswerfen von Kettenbestandteilen (s. Fig. 18) bewirkt über einen Hebelmechanismus 113 ein Auslenken der Hebel 4. Da dieses Auslenken jedoch blockiert ist, ist auch das Betätigen der Tasten 113a bei leerem Magazin blockiert.

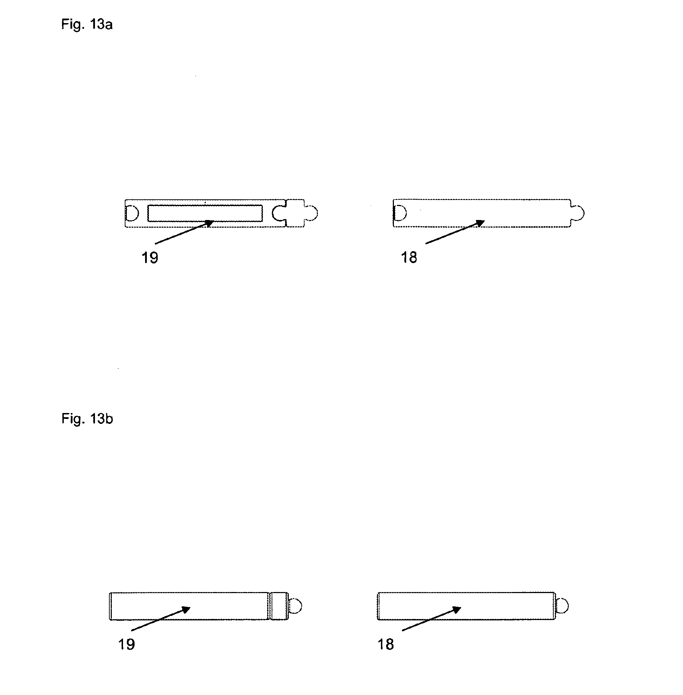

Fig. 13 zeigt zusammenfügbare Abstandshalter 18 und Strahlenquellen 19. Fig. 13a zeigt die Abstandshalter 18 und Strahlenquellen 19 im Querschnitt entlang einer Längsachse und Fig. 13b zeigt Abstandshalter 18 und Strahlenquelle 19 von außen betrachtet. Die Strahlenquellen 19 weisen dabei einen inneren radioaktiven Kern auf, in Fig. 13a als Rechteck dargestellt. Die Strahlenquellen 19 und Abstandshalter 18 weisen Enden auf, die so ausgebildet sind, dass sie zu einer Kette zusammengefügt werden können. Es können dabei auch zwei Abstandshalter 18 oder zwei Strahlenquellen 19 direkt zusammengefügt werden. Die Abstandshalter 18 bzw. Strahlenquellen 19 weisen bevorzugt jeweils männliche Enden (rechts) und weibliche Enden (links) auf. Durch die Ausbildung der Strahlenquellen 19 und Abstandshalter 18 sind die Ketten frei konfektionierbar.

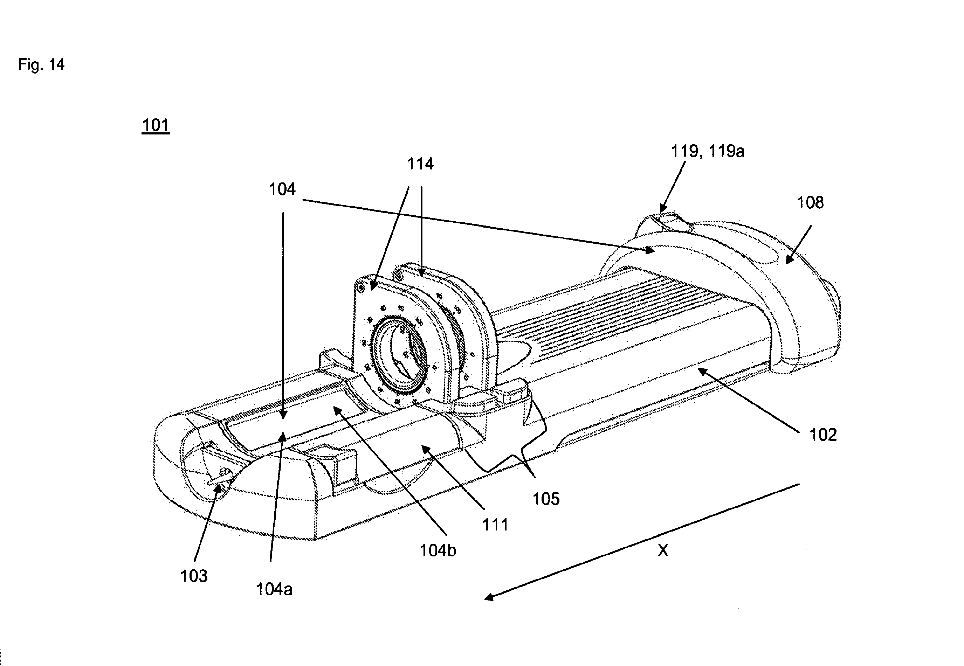

Fig. 14 zeigt nun eine perspektivische Ansicht eines erfindungsgemäßen Gerätes 101 zum Fügen und Konfektionieren von Strahlenketten mit radioaktiven Strahlenquellen, welches bevorzugt mit den oben beschriebenen Magazinen 1 betrieben wird.

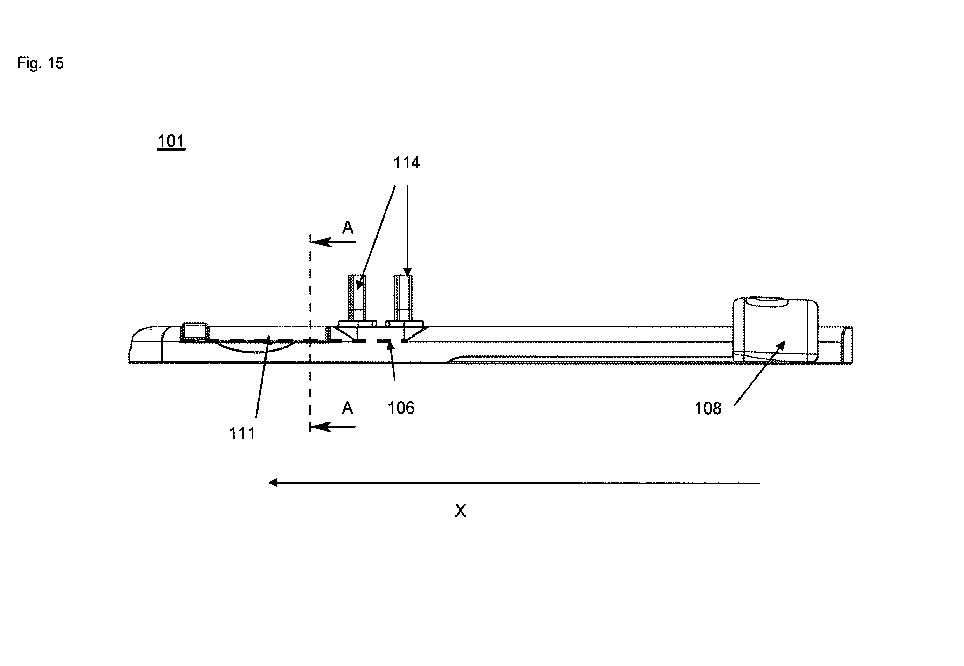

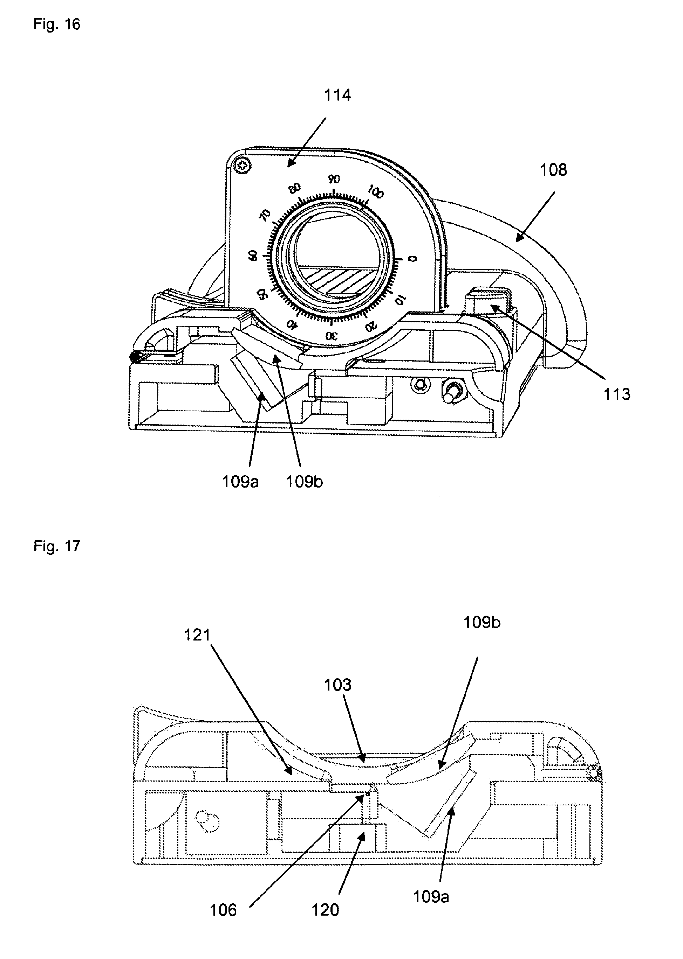

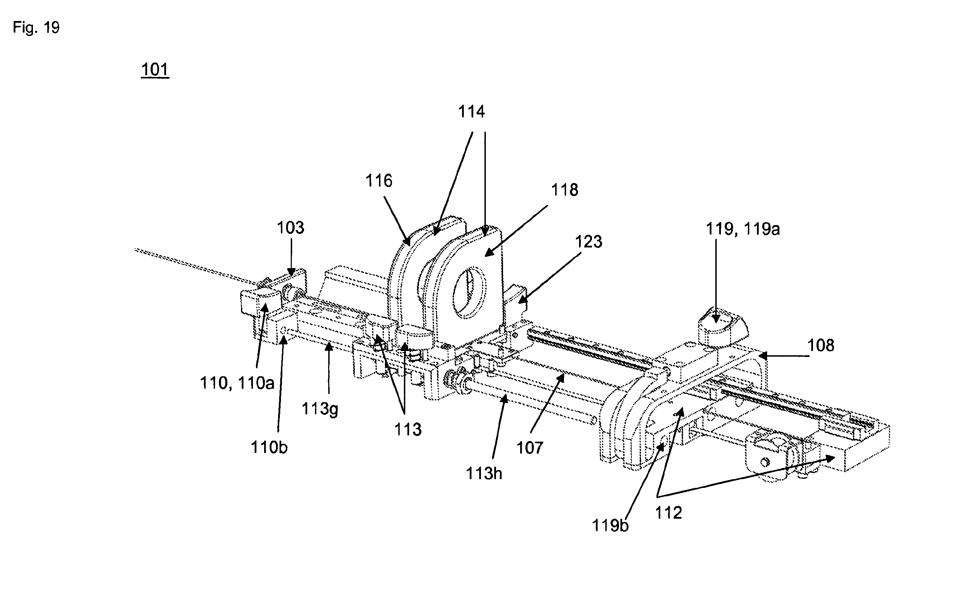

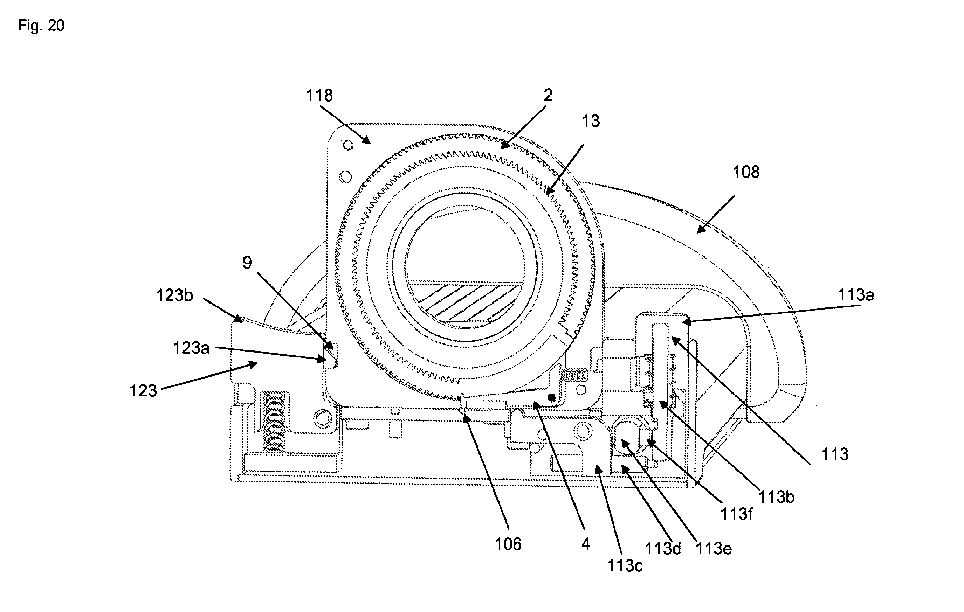

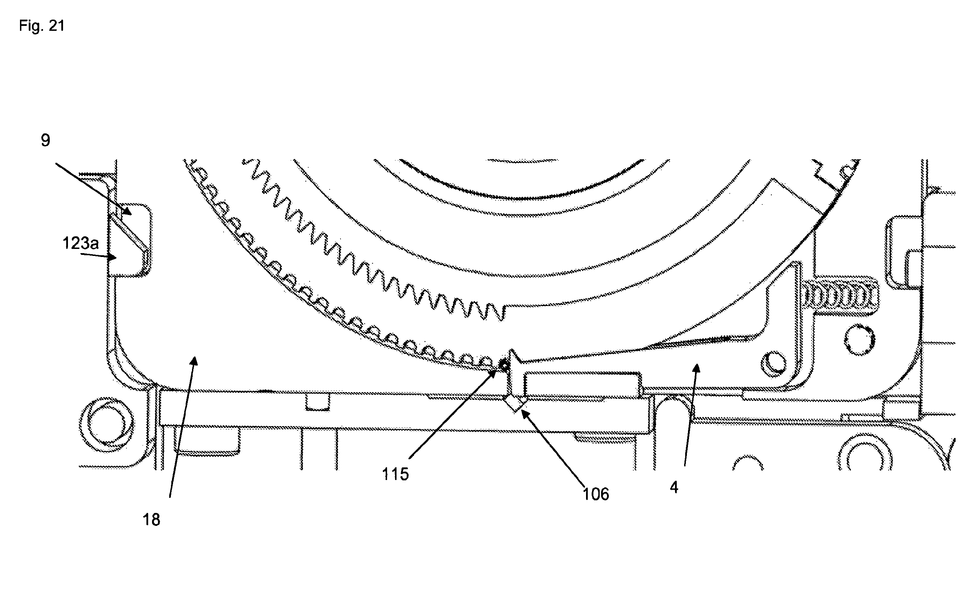

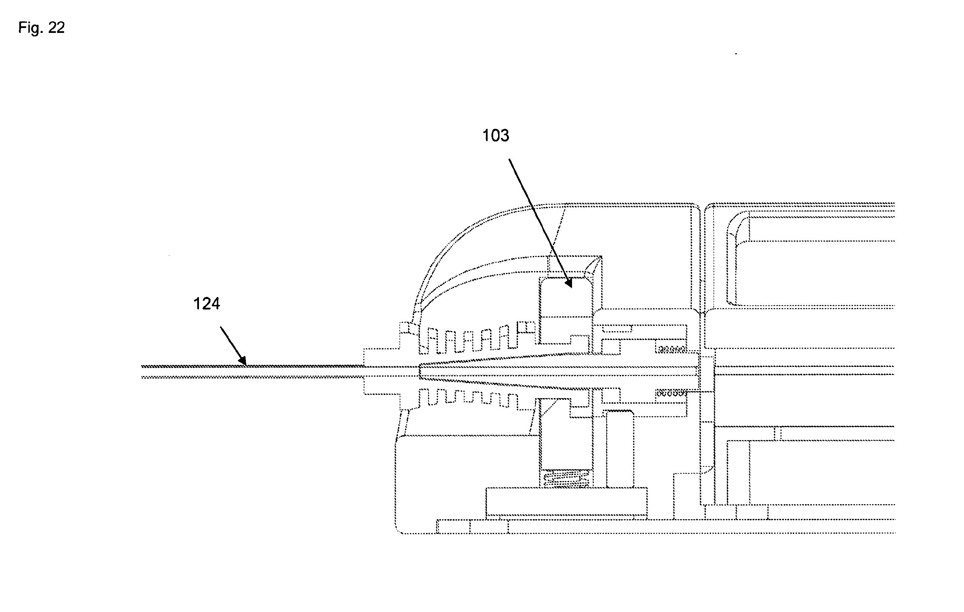

Fig. 15 zeigt eine Seitenansicht des erfindungsgemäßen Gerätes zum Fügen und Konfektionieren von Strahlenketten der Fig. 14. Fig. 16 ist ein perspektivischer Querschnitt durch das erfindungsgemäße Gerät entlang der Achse A-A in Fig. 15, gesehen entgegen der Richtung der Pfeile in Fig. 15. Fig. 17 zeigt einen Querschnitt durch das erfindungsgemäße Gerät entlang der Achse A-A in Fig. 15, gesehen in Richtung der Pfeile der Fig. 15. Fig. 18 ist eine erste Ansicht des erfindungsgemäßen Gerätes ohne Gehäuse. Fig. 19 ist eine zweite Ansicht des erfindungsgemäßen Gerätes ohne Gehäuse. Fig. 20 zeigt einen Querschnitt durch das Gerät zum Fügen durch ein Abstandshaltermagazin. Fig. 21 stellt eine Vergrößerung der Fig. 20 im unteren Bereich des Abstandshaltermagazins dar. Fig. 22 zeigt einen Längsschnitt durch den vorderen Bereich des erfindungsgemäßen Gerätes zum Fügen im Bereich des Nadelhalters.

Das erfindungsgemäß beschriebene Gerät 101 besteht wie in Fig. 14 gezeigt aus einem Gehäuse 102, einem Nadelhalter 103, einer Füge- und Sichteinheit 104 sowie einer Beladungseinheit 105. In die Beladungseinheit werden Magazine 114 für Kettenbestandteile eingesetzt. Die Füge- und Sichteinheit 104 umfasst bevorzugt einen Fügebereich 104a in dem die Kettenelemente zusammengefügt werden sowie eine Sichtbereich 104b zur Überprüfung der Anordnung der Kettenbestandteile. In einem bevorzugten Ausführungsbeispiel ist der Fügebereich 104a über eine Klappe 111 von außen erreichbar. An einem Ende des Gerätes ist der Nadelhalter 103 angeordnet, an dem anderen Ende ein Griff 108, der noch weiter erläutert werden wird. Am Griff 108 ist eine Sperre 119, ausgebildet als Tastelement angeordnet. Zwischen Griff 108 und Nadelhalter 103 sind die Magazine 114 und der Füge- bzw. Sichtbereich 104a, 104b angeordnet.

Die Aufgabe des Gerätes 101 besteht darin, mit seiner Hilfe radioaktive Strahlenquellen 115 und inaktive Abstandshalter 117 aus entsprechenden Magazinen 114 freizusetzen, diese mit Steckverbindungen versehenden Implantate (s. Fig. 13) oder Kettenbestandteile 115, 117 zu einer Kette zusammenzufügen, um mit diesen anschließend die dem Gerät 101 aufgesteckten Implantationsnadeln 124 zu befüllen (s. Fig. 22).

Zentrales Element des beschriebenen Gerätes 101 stellt der Arbeitskanal 106 dar, der sich wie in Fig. 15, 17, 19 und 20 gezeigt entlang der Achse X bzw. entlang einer Längesachse des Gerätes 101 ausdehnt. Der Arbeitskanal 106 beginnt vom Griff 108 aus gesehen bevorzugt kurz vor den Magazinhalterungen oder alternativ genau unterhalb des Auswurfs des dem Griff nächsten Magazins 114 und endet am Nadelhalter 113. Ein Beginn des Arbeitskanals 106 kurz vor den Magazinhalterungen ermöglicht eine bessere Führung des später zu beschreibenden Mandrels 107. Um den Arbeitskanal 106 herum sind die verschiedenen Einheiten angeordnet, gehalten durch das Gehäuse 102. Das Gehäuse 102 trägt und schützt die sonstigen Funktionseinheiten des Gerätes 101. Von rechts nach links ist in Fig. 14 und Fig. 15 zunächst der Griff 108, die Magazine 114 mit der Beladungseinheit 105, der Füge- und Sichtbereich 104a, 104b und der Nadelhalter 103 angeordnet.

Die Füge- und Sichteinheit 104 umfasst den Arbeitskanal 106 und beinhaltet einen Mandrel 107 (s. Fig. 18 oder 19), der über einen äußeren Griff 108 im Arbeitskanal 106 linear geführt ist sowie eine Spiegel-Linsen-Einheit 109 (s. Fig. 17) zur Visualisierung der Implantate im Fügebereich 104a. Der Mandrel 107 transportiert über den Griff 108, der durch den Anwender lateral verschoben werden kann, die aus dem Magazin freigegebenen Strahlenquellen 115 bzw. Abstandshalter 117 aus dem Teil des Arbeitskanales 106 unter den Magazinen 114 in den Fügebereich 104a. Im Fügebereich 104a kann die Konfiguration vorteilhafterweise über die Sichteinheit 104b überprüft werden. Nachdem die gewünschte Konfiguration der Implantate erstellt ist, werden diese im Fügebereich 104a mit Hilfe des Mandrel 107 zusammengeschoben und so zu einer Seed-Spacer-Kette gefügt. Als Gegenlager blockiert eine erste Sperre 110, die einen Taster 110a umfasst, während des Fügens den Übergang von Arbeitskanal 106 zum Nadelhalter 103. Nach dem Fügen wird die erste Sperre 110 geöffnet und erlaubt so das Ausschieben der gefügten Implantatkette über den Nadelhalter 103 in die Nadel 124 (s. Fig. 22). Die erste Sperre 110 ist hierbei bevorzugt vor dem Nadelhalter 103 und hinter dem Sichtbereich 104b angeordnet, kann aber auch mit dem Nadelhalter 103 zusammenfallen.

Der manuell bedienbare Nadelhalter 103 besteht aus einer Verschlusseinrichtung und einem Nadeladapter, der die gefügte Implantatkette in die aufgesteckte Nadel 124 leitet. Die Verschlusseinrichtung des Nadelhalters hält und sichert die Nadel 124 gegen Verlust beim Befüllen.

Ein Linsen-Spiegel-System 109 erlaubt einen indirekten und somit strahlungsgeschützten Sichtkontakt zu den Implantaten bzw. Kettenbestandteilen. Über eine Klappe 111 in der Sichteinheit 104b kann bei Bedarf direkt in den Arbeitskanal 106 eingegriffen werden, um so eventuelle Korrekturen an der Seed-Spacer-Konfiguration vorzunehmen. Für eine kurzzeitige Zwischenlagerung der Strahlenquellen bzw. Abstandshalter während einer Korrektur stehen unterhalb der strahlengeschützten Klappe 111 zwei muldenförmigen Lagerplätze zur Verfügung.

Beide Magazine 114 sind während des Betriebes im Gerät 101 über einen Sicherungsmechanismus 123 wie in Fig. 20 gezeigt einrastend fixiert und gegen ein Herausfallen gesichert. Der Sicherungsmechanismus 123 umfasst hierbei eine Nase, die in eine Sicherungsnut 9 eines Abstandshaltermagazins 18 eingreift. Die Magazine 114 können jedoch jederzeit durch Betätigen eines entsprechenden Auslösehebels entriegelt und entnommen werden. Ein Vertauschen der Magazine 114 in ihren bestimmungsgemäßen Steckplätzen ist durch eine mechanische Kodierung nicht möglich. So umfasst bevorzugt das Strahlenquellenmagazin 116 eine Kodierung, die von der Kodierung des Abstandshaltermagazins 118 verschieden ist. Die Kodierung kann durch einen Kodierungsdorn erfolgen, der in eine entsprechende Kodierungsöffnung 11 des Magazins eingreift. Die Kodierung des Strahlenquellenmagazins 116 verschiebt beim Einsetzen des Magazins 116 noch zusätzlich einen o.g. Verschlussschieber 5. Dieser schützt zusätzlich vor einer Strahlungsexposition beim Umgang und während des Transports des Magazins 116 vor dem Einsetzen in das Gerät 101. Nach Entnahme des Strahlenquellenmagazins 116 schließt auch wieder der Verschlussschieber 5.

Um Beschädigungen an den Implantaten durch zu große Kräfte beim Fügen der Implantate zu vermeiden, ist die Fügeeinheit 104 mit einer Magnetkupplung 122 versehen. Hierzu ist der den Griff 108 und den Mandrel 107 führende Schlitten 112 zweigeteilt ausgebildet, wie in Fig. 18 und Fig. 19 gezeigt. Beide Teile trennen sich bei Überbelastung der Haftkraft der Magnetkraft voneinander, womit ein weiteres Betätigen eines Hebels 119 wirkungslos bleibt.

Die Beladungseinheit 105 beinhaltet die Steckplätze für ein Abstandshalter- und ein Strahlenquellenmagazin 118, 116 sowie einen mechanischen Hebelmechanismus 113 zum Freisetzen der Implantate. Nach Einsetzen eines Magazins 114 und Betätigung einer entsprechenden Taste 113a im Hebelmechanismus 113 wird ein Implantat aus dem Magazin in den darunterliegenden Kanal 106 freigesetzt. Der Hebelmechanismus 113 pro Magazin 114 besteht aus einem Taster 113a, der bevorzugt gleitgelagert ist, einer Tasterstange 113b mit einer Quernut, wobei die Tasterstange 113b bevorzugt federnd gelagert und einseitig, zum ersten Federdruckstück 113d hin abgeflacht ist. Ferner umfasst der Hebelmechanismus 113 einem im Gehäuse rotatorisch und federnd gelagerten Hebel 113c mit einem erstem Federdruckstück 113d und einer quer zu den Tasterstangen 113b translatorisch geführten Schubstange 113e mit jeweils einem zweitem Federdruckstück 113f für jeden der Hebelmechanismen sowie eine erste Verlängerung 113g der Schubstange und eine zweite Verlängerung 113h der Schubstange.

Der Hebelmechanismus 113 wird im Folgenden unter Bezug auf die Figuren 18-20 näher erläutert werden. Durch Drücken auf den Taster 113a wird die bevorzugt beidseitig gleitlagergeführte Tasterstange 113b nach unten bewegt (s. Fig. 20). Hier drückt sie mit ihrem freien Ende auf den federnd gelagerten Zapfen des ersten Federdruckstückes 113d, wodurch das Federdruckstück 113d nach unten gedrückt wird. Da dieses Federdruckstück 113d direkt mit dem im Gehäuse drehbar gelagerten Hebel 113c verbunden ist, überträgt sie diese Bewegung direkt auf den Hebel 113c. Der Hebel 113c hebt dadurch sein dem Federdruckstück 113d gegenüberliegendes Ende, welches in das darüber angeordnete Magazin eingreift und hierdurch die Freigabe eines Implantates auslöst.

Um den Taster 113a nach dem Betätigen in der unteren Position zu fixieren, rastet ein zweites Federdruckstück 113f der Schubstange 113e in eine Quernut der abgeflachten Tasterstange 113b ein. Gleichzeitig gleitet der federnde Zapfen des ersten Federdruckstückes 113d des Hebels 113c an der abgeflachten Tasterstange 113b ab. Da die Hebel 113c federnd gelagert sind, stellen sie sich dadurch in ihre Ursprungslage zurück. Zweck dieses Mechanismus ist es, aus Sicherheitsgründen den Taster 113a nach Betätigung temporär in der gedrückten Position zu fixieren, jedoch den Hebel 113c direkt nach Auslösen des Magazins 114 in seine Ursprungslage zurückkehren zu lassen.

Wie in Fig. 18 und 19 zu sehen, ist die Schubstange 113e zu beiden Seiten des Hebelmechanismus 113 verlängert, mit einer ersten Verlängerung 113g und einer zweiten Verlängerung 113h. Die erste Verlängerung 113g der Schubstange 113b ist zwischen den Magazinen 114 und der ersten Sperre 110 angeordnet und kann zur Öffnung der ersten Sperre 110 in eine Öffnung 110b der ersten Sperre 110 eingreifen und den Taster 110a der ersten Sperre 110 öffnen. Die zweite Verlängerung 113h der Schubstange 113b ist zwischen den Magazinen und dem Griff 108 angeordnet und greift in eine Öffnung 119b der dritten Sperre 119 ein.

Wurde unter den Magazinen 114 Implantate 115, 117 freigesetzt und die Taster 113a verbleiben in der unteren Stellung, muss erst der Griff 109 in X-Richtung verschoben werden. Wird der Griff in X-Richtung verschoben und ist die dritte Sperre 119 geschlossen, schiebt der Nutzer mit dem Griff 108 die dritte Sperre 119 gegen die eine zweite Verlängerung 113h und somit gegen die Schubstange 113e. In diesem Zustand ist die zweite Verlängerung 107 in der Öffnung 119b der dritten Sperre 119 fixiert und wird mit dem Griff 108 verschoben. Die zweiten Federdruckstücke 113f der Schubstange 113e werden dann aus den Quernuten der Tasterstange 113b geschoben und somit die Taster 113a entriegelt. Da mit der Bewegung des Griffes 108 auch der Mandrel 107 bewegt wird, wurden gleichzeitig mit dem Entriegeln der Taster 113a die Implantate aus dem Raum unter den Magazinen verschoben.

Ist die dritte Sperre 119 geöffnet, kann der Griff 108 und der Mandrel 107 in X-Richtung verschoben werden, ohne von der dritten Sperre 119 und der zweiten Schubstangenverlängerung 113h begrenzt zu werden. Diese Stellung wird verwendet, um die Implantatketten zu fügen und später in die Nadel 123 zu schieben. In dieser Position der dritten Sperre 119 können jedoch die Taster 113a nicht wieder entriegelt werden.

Wurde die erste Sperre 110 geöffnet, um die Implantatkette nach dem Fügen in die Nadel 123 zu schieben, wird sie in dieser Position fixiert, um Beschädigungen der Kette durch die zurückfallende Sperre 110 zu vermeiden. Sie wird erst durch einen Impuls der ersten Verlängerung 113g der Schubstange 113e über die Öffnung 110b wieder verschlossen, wenn der Schlitten mit der dritten Sperre 119 die zweite Verlängerung 113h verschiebt. Im geschlossenen Zustand dient die erste Sperre 110 als Gegenlager zum Fügen der Implantatketten aus den Einzelimplantaten.

Durch den Hebelmechanismus 113 kann somit ein Auswerfen von zwei Kettenbestandteilen übereinander vermieden werden. Allerdings können die Taster 113a unterschiedlicher Magazine 114 auch gleichzeitig ausgelöst werden, da sie beabstandet über dem Arbeitskanal 106 angeordnet sind. Vorteilhafterweise kann somit das Konfektionieren durch gleichzeitiges Drücken (oder kurz hintereinander) von Strahlenquellen- und Abstandshaltermagazintasten 113a beschleunigt werden.

Fig. 20 zeigen einen Querschnitt durch das erfindungsgemäße Gerät 101 in Höhe des Abstandshaltermagazins 118 durch dieses hindurch. Hinter dem Abstandshaltermagazin 118 ist der Griff 108 sichtbar. Auf Höhe des Abstandshaltermagazins 118 befindet sich der Sicherungsmechanismus 123 der Aufnahmeeinrichtung für Magazine des Geräts 101. Der Sicherungsmechanismus 123 umfasst einen Vorsprung 123a, in Fig. 20 exemplarisch an seiner rechten oberen Seite angeordnet, der in die Sicherungsnut 9 des Abstandshaltermagazins 118 eingreift und das Magazin 118 im Gerät 101 gegen ein Herausfallen sichert. Der Sicherungsmechanismus 123 umfasst ferner eine Taste 123b im oberen Bereich. Durch Drücken der Taste 123b wird der Vorsprung 123a des Sicherungsmechanismus aus der Sicherungsnut 9 herausgeschwenkt und gibt das Magazin 118 frei. In Fig. 20 ist der Sicherungsmechanismus 123 auf einer Seite des Abstandshaltermagazins 118 angeordnet, der Auswerf-Hebelmechanismus 113 auf der anderen Seite. Der Auswerf-Hebelmechanismus 113 umfasst ebenfalls eine Taste 113a, die in Wirkverbindung mit dem Auswerferhebel 4 des Magazins 118 steht. Durch Drücken der Taste 113a des Hebelmechanismus 113 wird der Auswerferhebel 4 nach oben rotiert und ein Abstandshalter 119 nach unten in den Arbeitskanal 106 freigegeben. Fig. 21 zeigt die Anordnung des Arbeitskanals 106 der Fig. 20 in einer Vergrößerung. Der Arbeitskanal 106 hat bevorzugt eine V-Form, so dass die Kettenbestandteile im tiefsten Punkt des V's gelagert und geführt werden.

Fig. 22 zeigt eine Nadel 124, die in den Nadelhalter 103 des Gerätes 101 eingesteckt ist. Die Kettenbestandteile werden aus der Füge- und Sichteinheit in die Nadel 124 mittels des Mandrels 107 geschoben.

Es soll im Folgenden noch einmal in kurzen Worten die Reihenfolge der Betätigung der Elemente des Gerätes 101 zum Zusammenfügen von Kettenbestandteilen erläutert werden:
- Betätigen des Hebelmechanismus 113 und Auswerfen eines Kettenbestandteils; Blockierung des Hebelmechanismus 113 des betätigten Magazins;
- Verschiebung des Griffs 108 zur Bewegung des Mandrels 107 entlang der Gerätelängsachse im Arbeitskanal 106 zur Verschiebung des ausgeworfenen Kettenbestandteils in den Füge- und Sichtbereich 104a, 104b; Freigabe des zuvor blockierten Hebelmechanismus 113;
- Wiederholung von Schritt 1 und 2 bis die benötigten Kettenbestandteile freigegeben sind;
- Drücken und Einrasten der Taste 119a der der dritten Sperre 119 am Griff 108 zum Zusammenfügen der Kettenbestandteile durch Zusammendrücken der Kettenbestandteile mittels des Mandrels 107 gegen die erste Sperre 110;
- Freigabe der ersten Sperre 110 durch Drücken der Taste 110a ;
- Verschieben des Mandrels 107 zum Verschieben der zusammengefügten Kettenbestandteile in eine Nadel 124.
federngetriebenes oder manuelles Zurückfahren des Griffes 108 in seine Ursprungslage; erst nach erneutem Verschieben des Mandrel 107 wird die erste Sperre 110 wieder geschlossen, bevorzugt über den Eingriff der ersten Verlängerung 113g in die erste Öffnung 110b der ersten Sperre 110. Zusammenfassend ist festzuhalten, dass die vorliegende Erfindung ein vollsterilisierbares Gerät 101 zur Erzeugung von Strahlenquellen-Abstandshalter-Ketten offenbart. Das Gerät 101 benötigt keine elektromechanischen Bauteile. Die Anordnung der Strahlenquellen und Abstandshalter 115, 117 in den Ketten kann entsprechend eines patientenbezogenen Behandlungsplanes individuell und variabel konfiguriert erfolgen.

Zur Erzeugung der Strahlenquellen-Abstandshalter-Kette benötigt das beschrieben Gerät 101 lediglich zwei Magazine 116, 118, wodurch Verwirrungen bei der Zusammenstellung der Kette minimiert werden. Die Magazine 116, 118 sind in einer Reihe entlang der Gerätelängsachse angeordnet. Dabei entsteht ein für den Nutzer übersichtliches und anwenderfreundliches Erscheinungsbild.

Durch die beschriebene Kodierung von Gerät 101 und Magazin 116, 118 ist es unmöglich, das Strahlenquellen- bzw. das Abstandshalter-Magazin beim Einsatz in das Gerät 101 zu vertauschen.

Beide Magazine 116, 118 transportieren die bevorrateten Implantate in einen unter ihnen angeordneten und an dieser Stelle nach oben hin offenen Arbeitskanal 106. In diesem Kanal 106 werden die Implantate in den vorderen Bereich des Gerätes 101 verschoben, wo sie später zum sogenannten Strand gefügt werden. Dieses Prinzip verhindert, dass die Implantate aus dem hinteren Magazin 118 durch das vordere Magazin 116 geführt werden müssen, wodurch es zum Klemmen kommen könnte. Darüber hinaus verhindert dieses Prinzips Beschädigungen des Mandrels 107, falls ein Magazin 116, 118 versehentlich zu früh aus dem Gesamtaufbau entnommen wird. Der Abwurf der Implantate in nur einen einzigen Arbeitskanal 106 zum Abtransport und Fügen beider Kettenglieder minimiert die Gefahr eines Verbiegens des Mandrels 107.

Aufgrund dieser Anordnung von Magazinen 116, 118 zum Arbeitskanal 106 ist es nicht nötig, die Magazine 116, 118 zum Wechsel des auszuwerfenden Implantats in einem Winkel relativ zur Gerätelängsachse zu verschieben.

Die Magazine enthalten bevorzugt mehr als 50 und bis zu 100 Kettenbestandteile, die in einer Karussellanordnung platzsparend bevorratet sind. Es können aber auch mehr als 100 Kettenbestandteile in den Magazinen gelagert werden. Wegen des Inhalts von bevorzugt ca. 100 Strahlenquellen bzw. 100 Abstandshaltern werden sich die meisten Strahlenquellenbehandlungen mit nur jeweils einem Magazin 116, 118 durchführen lassen.

Durch Sperren ist das Magazin 116, 118 nicht mehr auslösbar, wenn der Strahlenquellen- bzw. Abstandshalter-Vorrat verbraucht ist.

Der Antrieb der Magazine 116, 118 erfolgt durch die Verwendung einer Konstantkraftfeder.3 zu jeder Zeit mit der gleichen Kraft. Die Hemmung dieser Konstantkraftfeder 3 wird durch ein von außen angetriebenes Ankersystem 4 erzeugt.

Die Magazine 116, 118 sind nach der Behandlung wiederverwendbar. Sie beinhalten keine elektromechanischen Bauteile.

Die Anzahl der in den Magazinen verbliebenen Strahlenquellen und Abstandshaltern 115, 117 ist jederzeit an den Magazinen 116, 118 ablesbar. Daher ist kein Zählen der verbrauchten Strahlenquellen und Abstandshalter 115, 117 während und nach der Behandlung mehr nötig.

Die Magazine 116, 118 bieten aufgrund ihres nach allen Seiten abschirmenden Gehäuses 102 einen optimalen Strahlenschutz. Erst nach dem Einsetzen des Magazins 116, welches die aktiven Strahlenquellen 115 beinhaltet, wird die Öffnung 10 des Strahlenquellenmagazins 116 freigegeben. Bei Entnahme des Magazins 116 wird die Öffnung 10 wieder geschlossen. Der Strahlenschutz ist daher über den gesamten Zeitraum der Anwendung und während des Transportes, d.h. auch außerhalb des Geräts gewährleistet. Bei dem Magazin der nichtaktiven Abstandshalter 117 kann auf dieses Funktionsdetail verzichtet werden.

Die individuell an den Patienten angepasste Strahlenquellen-Abstandshalter-Kettenkonfiguration vermindert im Gegensatz zu vorgefertigten Strahlenquellen-Abstandshalter-Ketten freien Strahlenquellen-Abfall. Nicht verwendete Strahlenquellen 115 und Abstandshalter 117 verbleiben in den entsprechenden Magazinen 116, 118. Eine Strahlenexposition kann daher nicht auftreten.

Darüber hinaus wird eine Strahlenexposition durch die freigesetzten Strahlenquellen im Arbeitskanal 106 durch eine Abschirmung und eine indirekte Draufsicht durch einen Spiegel verhindert.

### Bezugszeichenliste

- 1: Magazin
- 2: Mittel zur Aufnahme von Kettenbestandteilen oder Seedablage
- 3: Erste Spannfeder - Konstantkraftfeder
- 4: Auswerferhebel
- 5: Verschlussschieber
- 6: Vertiefungen
- 7: Magazingehäuse
- 7a: Gehäusedeckel
- 7b: Gehäuseschale
- 8: Innerer Lagerring
- 9: Sicherungsnuten
- 10: Auswurf
- 11: Kodierungsbohrung
- 12: Öffnung
- 13: Oberer Zahnkranz
- 14: Anzeige
- 15: Markierung
- 16: Innere Wand
- 17: Zentrale Öffnung
- 18: Abstandshalter
- 19: Strahlenquellen
- 20: Erste Druckfeder - für den Auswerferhebel 4
- 21: Zweite Druckfeder - für den Verschlussschieber 5
- 22: Blockierelement der Auslösung bei leeren Magazinen

- 101: Gerät zum Fügen von Strahlenquellen
- 102: Gerätegehäuse
- 103: Nadelhalter
- 104: Füge- und Sichteinheit
- 104a: Fügebereich
- 104b: Sichtbereich
- 105: Beladungseinheit
- 106: Arbeitskanal
- 107: Mandrel
- 108: Äußerer Griff
- 109: Spiegel-Linsen-Einheit
- 109a: Spiegel
- 109b: Linse
- 110: Erste Sperre - Sperre im Arbeitskanal zum Fügen der Kettenbestandteile
- 110a: Taster der ersten Sperre
- 110b: Aufnahmeöffnung für erste Verlängerung der Schubstange 113g
- 111: Klappe
- 112: Schlitten
- 113: Hebel-Mechanismus
- 113a: Taste des Hebel-Mechanismus
- 113b: Tasterstange
- 113c: Hebel
- 113d: Erstes Federdruckstück
- 113e: Schubstange
- 113f: Zweites Federdruckstück
- 113g: Erste Verlängerung der Schubstange 113e
- 113h: Zweite Verlängerung der Schubstange 113h
- 114: Magazin
- 115: Strahlenquelle
- 116: Strahlenquellenmagazin
- 117: Abstandshalter
- 118: Abstandshaltermagazin
- 119: Dritte Sperre - Sperre im Griff
- 119a: Taster der dritten Sperre
- 119b: Aufnahmeöffnung für zweite Verlängerung der Schubstange 113h
- 120: Bauraum für Beleuchtung
- 121: Öffnung der Beladungseinheit zum Arbeitskanal
- 122: Magnetkupplung
- 123: Sicherungsmechanismus Magazine
- 123a: Vorsprung des Sicherungsmechanismus 123
- 123b: Taste des Sicherungsmechanismus 123
- 124: Nadel

## Patentansprüche

1. Gerät (101) zum Zusammenfügen von Kettenbestandteilen zu einer Kette, wobei mindestens ein Kettenbestandteil aus radioaktiven Strahlenquellen (115) besteht, umfassend:
ein Gehäuse (102),
einen Arbeitskanal (106), der sich entlang einer ersten Achse (X) des Gehäuses erstreckt,
eine Beladungseinheit (105), verbunden mit dem Arbeitskanal (106) und umfassend mindestens zwei Aufnahmeeinrichtungen für Magazine von Kettenbestandteilen, wobei mindestens eine Aufnahmeeinrichtung geeignet ist, ein Strahlenquellenmagazin (116) aufzunehmen, sowie mindestens ein Mittel (113) zum Auswerfen der Kettenbestandteile aus den aufgenommenen Magazinen, und
eine Fügeeinheit (104) zum Zusammenfügen von Kettenbestandteilen,
**dadurch gekennzeichnet, dass**
die mindestens zwei Aufnahmeeinrichtungen derart angeordnet sind, dass in ihnen aufgenommene Magazine für Kettenbestandteile beabstandet entlang der ersten Achse (X) vertikal über dem Arbeitskanal (106) angeordnet sind.

2. Gerät (101) nach Anspruch 1, wobei die zweite Aufnahmeeinrichtung eingerichtet ist, ein Abstandshaltermagazin (118) mit Abstandshaltern (117) aufzunehmen.

3. Gerät (101) nach Anspruch 1 oder 2, wobei die Fügeeinheit (104) einen Fügebereich (104a) und eine Sichteinheit (104b) umfasst, so dass ein Zusammenfügen der Kette von außen betrachtbar ist.

4. Gerät (101) nach Anspruch 3, wobei die Sichteinheit (104b) eine indirekte Sichteinheit ist, so dass die Kette indirekt betrachtbar ist.

5. Gerät (101) nach einem der vorhergehenden Ansprüche, ferner umfassend eine Klappe (111) zum Öffnen des Gehäuses (102) im Fügebereich (1 04a) der Fügeeinheit (104).

6. Gerät (101) nach einem der vorhergehenden Ansprüche, wobei ferner ein Nadelhalter (103) an einem Ende des Arbeitskanals (106) angeordnet ist und zwischen Nadelhalter (103) und Fügeeinheit (104) eine erste Sperre (110) angeordnet ist.

7. Gerät (101) nach einem der vorhergehenden Ansprüche, wobei die Fügeeinheit (104) ferner einen Mandrel (107) umfasst, der im Arbeitskanal (106) entlang der ersten Achse (X) verschiebbar ausgebildet ist.

8. Gerät (101) nach Anspruch 7, wobei der Mandrel (107) über einen an dem Gehäuse (102) verschiebbar gelagerten äußeren Griff (108) verschiebbar ausgebildet ist.

9. Gerät (101) nach Anspruch 7 oder 8, wobei die Fügeeinheit (104) ferner eine Magnetkupplung (122) umfasst, derart, dass der äußere Griff (108) und der Mandrel (107) bei Überschreitung eines Kraftschwellenwertes voneinander trennbar ausgebildet sind.

10. Gerät (101) nach einen der Ansprüche 7 bis 9, wobei der Mandrel (107) und das mindestens eine Mittel zum Auswerfen von Kettenbestandteilen (113) miteinander über eine zweite Sperre derart gekoppelt sind, dass nach einmaliger Betätigung des Mittels zum Auswerfen (113) keine zweite Betätigung des Mittels zum Auswerfen (113) erfolgen kann, bevor eine Verschiebung des Mandrels (107) vorgenommen wurde.

11. Gerät (101) nach einem der vorhergehenden Ansprüche, wobei die Aufnahmeeinrichtungen zwei Sicherungsmechanismen (123) zum Einrasten, Sichern und Herauslösen von Magazinen mit Kettenbestandteilen im Gehäuse (102) umfassen.

12. Gerät (101) nach einem der vorhergehenden Ansprüche, ferner umfassend ein dritte Sperre (119), die im verriegelten Zustand eine Verschiebung des Mandrels (107) nur bis zu einem Punkt ermöglicht, bei dem ein Zusammenfügen der Kettenbestandteile im Arbeitskanal (106) noch nicht erfolgt.

13. Gerät (101) nach Anspruch 12, wobei die dritte Sperre (119) mit der ersten Sperre (110) zwischen Nadelhalter (103) und Fügeeinheit (104) verknüpft ist, so dass nur mit gelöster dritter Sperre (119) auch die erste Sperre (110) im Nadelhalter (103) entriegelbar ist.

14. Gerät (101) nach einem der vorhergehenden Ansprüche, wobei die Aufnahmeeinrichtungen Elemente zur Kodierung enthalten, die mit entsprechenden Kodierungselementen eines Magazins zusammenwirkbar ausgebildet sind, so eine Aufnahmeeinrichtung nur für eine spezielle Art von Magazin verwendbar ist.

15. System zum Zusammenfügen von Kettenbestandteilen zu einer Kette mit radioaktiven Strahlenquellen (115) besteht, umfassend:
ein Gehäuse (102),
einen Arbeitskanal (106), der sich entlang einer ersten Achse (X) des Gehäuses erstreckt,
eine Beladungseinheit (105), verbunden mit dem Arbeitskanal (106) und umfassend mindestens zwei Aufnahmeeinrichtungen für Magazine von Kettenbestandteilen sowie mindestens ein Mittel (113) zum Auswerfen der Kettenbestandteile aus den aufgenommenen Magazinen,
ein erstes Magazin (116) für radioaktive Strahlenquellen (115), angeordnet in einer der mindestens zwei Aufnahmeeinrichtungen,
ein zweites Magazin (118) für weitere, von den Strahlenquellen (115) verschiedene Kettenbestandteile (117), angeordnet in der anderen der mindestens zwei Aufnahmeeinrichtungen,
eine Fügeeinheit (104) zum Zusammenfügen von Kettenbestandteilen,
**dadurch gekennzeichnet, dass**
der Arbeitskanal (106) nicht durch die Magazine (116, 118) hindurchführt, und
das erste Magazin (116) für radioaktive Strahlenquellen (115) und das zweite Magazin (118) für weitere Kettenbestandteile (117) beabstandet entlang der ersten Achse (X) vertikal über dem Arbeitskanal (106) derart angeordnet sind, dass Kettenbestandteile nach unten in den Arbeitskanal (106) ausgeworfen werden.

## Claims

1. A device (101) for joining together chain components to form a chain, wherein at least one chain component consists of radioactive radiation sources (115), comprising:
a housing (102),
a work channel (106) that extends along a first axis (X) of the housing,
a loading unit (105) connected with the work channel (106) and comprising at least two receiving devices for chain component cartridges, wherein at least one receiving device is suitable for accommodating a radiation source cartridge (116), as well as at least one means (113) for ejecting the chain components from the accommodated cartridges, and
a joining unit (104) for joining together chain components,
**characterized in that**
the at least two receiving devices are arranged in such a way that chain component cartridges accommodated therein are spaced apart from each other and arranged along the first axis (X), vertically above the work channel (106).

2. The device (101) according to claim 1, wherein the second receiving device is set up to accommodate a spacer cartridge (118) with spacers (117).

3. The device (101) according to claim 1 or 2, wherein the joining unit (104) encompasses a joining area (104a) and a display unit (104b), so that the process of joining the chain can be viewed from outside.

4. The device (101) according to claim 3, wherein the display unit (104b) is an indirect display unit, so that the chain can be viewed indirectly.

5. The device (101) according to one of the preceding claims, further encompassing a flap (111) for opening the housing (102) in the joining area (104a) of the joining unit (104).

6. The device (101) according to one of the preceding claims, wherein a needle holder (103) is further arranged at one end of the work channel (106), and a first barrier (110) is arranged between the needle holder (103) and joining unit (104).

7. The device (101) according to one of the preceding claims, wherein the joining unit (104) further encompasses a mandrel (107) designed to slide in the work channel (106) along the first axis (X).

8. The device (101) according to claim 17, wherein the mandrel (107) is designed to slide via an external handle (108) slidably mounted on the housing (102).

9. The device (101) according to claim 7 or 8, wherein the joining unit (104) further encompasses a magnetic coupling (122) in such a way that the external handle (108) and mandrel (108) can be separated from each other when a force threshold is exceeded.

10. The device (101) according to one of claims 7 to 9, wherein the mandrel (107) and the at least one means for ejecting chain components (113) are coupled with each other by means of a second barrier in such a way that, after the ejection means (113) has been activated once, the ejection means (113) cannot be activated again before the mandrel (107) has been shifted.

11. The device (101) according to one of the preceding claims, wherein the receiving devices encompass two securing mechanisms (123) for latching, securing and detaching chain component cartridges in the housing (102).

12. The device (101) according to one of the preceding claims, further encompassing a third barrier (119), which when locked allows the mandrel (107) to shift only to a point at which the chain components in the work channel (106) are not yet being joined together.

13. The device (101) according to claim 12, wherein the third barrier (119) is linked with the first barrier (110) between the needle holder (103) and joining unit (104), so that the first barrier (110) in the needle holder (103) can also be unlatched only with the third barrier (119) detached.

14. The device (101) according to one of the preceding claims, wherein the receiving devices contain elements for coding purposes, which are designed to interact with corresponding coding elements of a cartridge, so that such a receiving device can only be used for a special type of cartridge.

15. A system for joining chain components comprising radioactive radiation sources (115), encompassing:
a housing (102),
a work channel (106) that extends along a first axis (X) of the housing,
a loading unit (105) connected with the work channel (106) and comprising at least two receiving devices for chain component cartridges, as well as at least one means (113) for ejecting the chain components from the accommodated cartridges,
a first cartridge (116) for radioactive radiation sources (115), arranged in one of the at least two receiving devices ,
a second cartridge (118) for additional chain components (117) differing from the radiation sources (115), arranged in the other of the at least two receiving devices,
a joining unit (104) for joining together chain components,
**characterized in that**
the work channel (106) does not pass through the cartridges (116, 118), and that
the first cartridge (116) for radioactive radiation sources (115) and the second cartridge (118) for additional chain components (117) are spaced apart and
arranged along the first axis (X), vertically above the work channel (106) in such a way that chain components are downwardly ejected in the work channel (106).

## Revendications

1. Appareil (101) pour l'assemblage de composants de chaîne en une chaîne, dans lequel au moins un composant de chaîne est constitué de sources de rayonnement radioactives (115), comprenant :
- un boîtier (102),
- un canal de travail (106) s'étendant le long d'un premier axe (X) du boîtier,
- une unité de chargement (105) reliée au canal de travail (106) et comprenant au moins deux dispositifs d'accueil pour des magazines de composants de chaîne, dans laquelle au moins un dispositif d'accueil est adapté pour accueillir un magazine de sources de rayonnement (116), ainsi qu'au moins un moyen (113) pour l'éjection des composants de chaîne hors des magazines accueillis, et
- une unité d'assemblage (104) pour l'assemblage de composants de chaîne,
**caractérisé en ce que**
les au moins deux dispositifs d'accueil sont agencés de telle manière que les magazines ainsi accueillis pour les composants de chaîne sont disposés verticalement au-dessus du canal de travail (106) et espacés le long du premier axe (X).

2. Appareil (101) selon la revendication 1, dans lequel le deuxième dispositif d'accueil est conçu pour accueillir un magazine d'espaceurs (118) avec des espaceurs (117).

3. Appareil (101) selon la revendication 1 ou 2, dans lequel l'unité d'assemblage (104) comprend une zone d'assemblage (104a) et une unité de vision (104b), de sorte qu'un assemblage de la chaîne peut être observé de l'extérieur.

4. Appareil (101) selon la revendication 3, dans lequel l'unité de vision (104b) est une unité de vision indirecte, de sorte que la chaîne peut être observée indirectement.

5. Appareil (101) selon l'une des revendications précédentes, comprenant en outre un clapet (111) pour l'ouverture du boîtier (102) dans la zone d'assemblage (104a) de l'unité d'assemblage (104).

6. Appareil (101) selon l'une des revendications précédentes, dans lequel un support d'aiguille (103) est en outre monté sur une extrémité du canal de travail (106), et un premier verrouillage (110) est installé entre le support d'aiguille (103) et l'unité d'assemblage (104).

7. Appareil (101) selon l'une des revendications précédentes, dans lequel l'unité d'assemblage (104) comprend en outre un mandrin (107) formé dans le canal de travail (106) de façon coulissante le long du premier axe (X).

8. Appareil (101) selon la revendication 7, dans lequel le mandrin (107) est conçu de façon à pouvoir coulisser au-dessus d'une poignée extérieure (108) montée de façon coulissante sur le boîtier (102).

9. Appareil (101) selon la revendication 7 ou 8, dans lequel l'unité d'assemblage (104) comprend en outre un accouplement magnétique (122), de sorte que la poignée extérieure (108) et le mandrin (107) peuvent être séparés l'un de l'autre lors d'un dépassement d'une valeur seuil d'une force.

10. Appareil (101) selon l'une des revendications 7 à 9, dans lequel le mandrin (107) et l'au moins un moyen pour l'éjection de composants de chaîne (113) sont accouplés de telle manière par un deuxième verrouillage, que suite à un actionnement unique du moyen pour l'éjection (113), un deuxième actionnement du moyen pour l'éjection (113) ne sera pas possible tant qu'un déplacement du mandrin (107) n'aura pas eu lieu.

11. Appareil (101) selon l'une des revendications précédentes, dans lequel les dispositifs d'accueil comprennent deux mécanismes de blocage (123) pour l'enclenchement, le blocage et le retrait de magazines avec des composants de chaîne dans le boîtier (102).

12. Appareil (101) selon l'une des revendications précédentes, comprenant en outre un troisième verrouillage (119) empêchant à l'état verrouillé le déplacement du mandrin (107) au-delà d'un certain point, auquel un assemblage des composants de chaîne dans le canal de travail (106) n'a pas encore lieu.

13. Appareil (101) selon la revendication 12, dans lequel le troisième verrouillage (119) est relié au premier verrouillage (110) entre le support d'aiguille (103) et l'unité d'assemblage (104), de sorte que le premier verrouillage (110) ne peut être déverrouillé dans le support l'aiguille (103) que lorsque le troisième verrouillage (119) est défait.

14. Appareil (101) selon l'une des revendications précédentes, dans lequel les dispositifs d'accueil contiennent des éléments pour le codage, conçus pour pouvoir coopérer avec des éléments de codage correspondants d'un magazine, de sorte qu'un dispositif d'accueil n'est utilisable que pour un certain type de magazine.

15. Système pour l'assemblage de composants de chaîne en une chaîne avec des sources de rayonnement radioactives (115), comprenant :
- un boîtier (102),
- un canal de travail (106) s'étendant le long d'un premier axe (X) du boîtier,
- une unité de chargement (105) reliée au canal de travail (106) et comprenant au moins deux dispositifs d'accueil pour des magazines de composants de chaîne, ainsi qu'au moins un moyen (113) pour l'éjection des composants de chaîne hors des magazines accueillis,
- un premier magazine (116) pour des sources de rayonnement radioactives (115), disposé dans l'un des au moins deux dispositifs d'accueil,
- un deuxième magazine (118) pour d'autres composants de chaîne (117) différents des sources de rayonnement (115), disposé dans l'autre des au moins deux dispositifs d'accueil,
- une unité d'assemblage (104) pour l'assemblage de composants de chaîne,
**caractérisé en ce que**
- le canal de travail (106) ne passe pas à travers les magazines (116, 118), et
- le premier magazine (116) pour les sources de rayonnement radioactives (115) et le deuxième magazine (118) pour les autres composants de chaîne (117) sont agencés verticalement au-dessus du canal de travail (106) et espacés le long du premier axe (X), de telle manière que les composants de chaîne sont éjectés vers le bas dans le canal de travail (106).
